# EUROPEAN PATENT APPLICATION

(11) **EP 4 454 758 A1**
(43) Date of publication of application: **30.10.2024**
(21) Application number: 23315134.9
(22) Date of filing: 28.04.2023
(51) Int. Cl.: B01L 99/00, G01N 21/00

(54) **NUCLEIC ACID AMPLIFICATION PROCESS CONTROLS**

(71) Applicant: Mobidiag Oy, 02150 Espoo (FI)
(72) Inventor: Hennetin, Jérôme, 75011 PARIS (FR); Bondet, Jean-Sebastien, 75011 PARIS (FR)
(74) Representative: Regimbeau

(57) **Abstract**

Disclosed are methods, systems, computer readable media of monitoring for the introduction of one or more liquids into a reaction chamber. Exemplary methods include determining fluorescence-based values from fluorophore-containing nucleic acid amplification reaction mixtures and determining whether those values satisfy predetermined thresholds and/or ranges or otherwise indicate abnormalities.

## Description

### FIELD

This disclosure relates to the field of nucleic acid amplification. More particularly, the disclosure concerns methods, materials, apparatuses, and systems for monitoring for the introduction of one or more liquids into a reaction chamber.

### INTRODUCTION

Nucleic acid amplification reactions are widely used in research and clinical laboratories for the detecting genetic disorders and infectious diseases. Nucleic amplification reactions utilize enzymes such as polymerases or ligases, separately or in combination, to generate multiple copies of a target nucleic acid sequence in primer extension reactions which incorporate nucleotides or by ligations of adjacent probes that are complementary to a target nucleic acid sequence. In such reactions, each template generates more copies, and the copies may themselves become templates. (The nucleic acid copies are referred to as "amplicons.") In a nucleic acid amplification reaction, a reaction mixture may be subjected to a number of thermal cycles ("PCR cycles"), each of which includes a denaturation step, and primer annealing step, and a primer extension step. In the denaturation step, a double-stranded DNA template molecule is made single-stranded; in the primer annealing step, primers bind to complementary sequences in the single-stranded DNA template; and in the primer extension step, new DNA strands are formed from the primers. A target nucleic acid sequence can be subjected to denaturation, annealing, and elongation conditions until there is a sufficient amount of amplicon in a reaction mixture to render the target nucleic acid sequence detectable or quantifiable. During the early stages of PCR, the amplification is exponential.

Nucleic acid amplification reactions rely on the accuracy of the buffer composition of the reaction mixtures. Amplification of the target nucleic acids is mainly performed by an enzymatic method using one or more polymerases such as DNA polymerase, reverse transcriptase, and/or RNA polymerase. These enzymes are sensitive to aspects of buffer composition, such as types of salt, salt concentrations, and pH. Nucleic acid amplification reactions commonly use a buffer such as Tris, a source of magnesium such as MgSO₄ or MgCl₂, and a salt of a monovalent cation such as KCl to maintain enzyme functionality.

Abnormalities in buffer volume and composition can arise during reaction mixture preparation and/or be caused by errors in the functionality of nucleic acid amplification systems. Such abnormalities can lead to unreliable or inconsistent results and even complete failure of nucleic acid amplification reactions. Existing methods may not indicate whether a nucleic amplification reaction is successful or has failed until after a reaction run is completed. The absence of amplification is a clear indication of failure but generally does not indicate the reason for the failure. Disclosed herein are materials, methods, and systems for detecting abnormalities that relate to detecting abnormalities in liquid filling of a reaction chamber. The methods disclosed herein can rely on the effect of the presence or concentration of nucleic acids, nucleotides, and/or fluorophores on the fluorescent properties of reaction mixtures.

### SUMMARY

The following embodiments are among those provided by the disclosure.

Embodiment 1 is a method of monitoring for the introduction of one or more liquids into a reaction chamber, the method comprising:
a) obtaining a first fluorescence measurement from the reaction chamber;
b) after step a), introducing, or attempting to introduce, a first liquid of the one or more liquids into the reaction chamber;
c) after step b), obtaining a second fluorescence measurement from the reaction chamber;
d) determining a first value from the first fluorescence measurement and the second fluorescence measurement, the first value indicating a degree of change in fluorescence in the reaction chamber resulting from step b); and
e) comparing the first value to a first predetermined threshold or range, wherein an abnormality is detected if the first value does not satisfy the first predetermined threshold or the first value is outside the first predetermined range.

Embodiment 2 is a system comprising: a docking station configured to receive a vessel comprising a reaction chamber;a fluorometer configured to measure fluorescence in the reaction chamber;and a processor operably linked to the fluorometer and a memory;
the memory comprising instructions that when executed by the processor cause the system to perform a method of monitoring for the introduction of one or more liquids into the reaction chamber, the method comprising:
a) obtaining a first fluorescence measurement from the reaction chamber;
b) after step a), introducing, or attempting to introduce, a first liquid of the one or more liquids into the reaction chamber;
c) after step b), obtaining a second fluorescence measurement from the reaction chamber;
d) determining a first value from the first fluorescence measurement and the second fluorescence measurement, the first value indicating a degree of change in fluorescence in the reaction chamber resulting from step b); and
e) comparing the first value to a first predetermined threshold or range, wherein an abnormality is detected if the first value does not satisfy the first predetermined threshold or the first value is outside the first predetermined range.

Embodiment 3 is a computer-readable medium comprising:
instructions that when executed by a processor of a system cause the system to perform a method of monitoring for the introduction of one or more liquids into a reaction chamber, the method comprising:
a) obtaining a first fluorescence measurement from the reaction chamber;
b) after step a), introducing, or attempting to introduce, a first liquid of the one or more liquids into the reaction chamber;
c) after step b), obtaining a second fluorescence measurement from the amplification reaction chamber;
d) determining a first value from the first fluorescence measurement and the second fluorescence measurement, the first value indicating a degree of change in fluorescence in the reaction chamber resulting from step b); and
e) comparing-the first value to a first predetermined threshold or range, wherein an abnormality is detected if the first value does not satisfy the first predetermined threshold or the first value is outside the first predetermined range.

Embodiment 4 is the method, system, or computer-readable medium of any one of the preceding embodiments, wherein introducing, or attempting to introduce, the first liquid into the reaction chamber comprises pumping, or attempting to pump, the first liquid through a tip into the reaction chamber, optionally wherein the tip is a needle tip or a pipette tip.

Embodiment 5 is the method, system, or computer-readable medium of any one of the preceding embodiments, wherein the vessel is a multi-chambered receptacle or the reaction chamber is contained within a multi-chambered receptacle.

Embodiment 6 is the method, system, or computer-readable medium of any one of the preceding embodiments, wherein the first value is determined as a ratio of the second fluorescence measurement to the first fluorescence measurement.

Embodiment 7 is the method, system, or computer-readable medium of any one of the preceding embodiments, wherein the first fluorescence measurement is an average or median of a plurality of individual measurements acquired in step a).

Embodiment 8 is the method, system, or computer-readable medium of any one of the preceding embodiments, wherein the second fluorescence measurement is an average or median of a plurality of individual measurements acquired in step c).

Embodiment 9 is the method, system, or computer-readable medium of any one of the preceding embodiments, wherein after step b) and before step c), a nucleic acid amplification reaction is initiated in the reaction chamber.

Embodiment 10 is the method, system, or computer-readable medium of the immediately preceding embodiment, wherein initiation of the nucleic acid amplification reaction comprises initiating a denaturation or reverse transcription step.

Embodiment 11 is the method, system, or computer-readable medium of the immediately preceding embodiment, wherein the second fluorescence measurement is obtained during the denaturation or reverse transcription step.

Embodiment 12 is the method, system, or computer-readable medium of any one of the preceding embodiments, wherein the reaction chamber is contained within a microfluidic cartridge.

Embodiment 13 is the method, system, or computer-readable medium of the immediately preceding embodiment, wherein the microfluidic cartridge comprises:
a plurality of functional areas comprising a sample preparation area, a nucleic acid amplification area, and a waste area;
a central distribution hub; and
a pump, a plurality of valves, and a fluidic network of microchannels connecting the functional areas to the hub;
wherein the pump, plurality of valves, and fluidic network of microchannels can drive movement of a fluid from a first functional area through the central distribution hub to a second functional area of the plurality of functional areas; and the reaction chamber is or is within the nucleic acid amplification area.

Embodiment 14 is the method, system, or computer-readable medium of the immediately preceding embodiment, wherein the first liquid is located in the nucleic acid amplification area during step c) if it was successfully introduced in step b).

Embodiment 15 is the method, system, or computer-readable medium of the immediately preceding embodiment, wherein the first liquid is located in the nucleic acid amplification area during step c).

Embodiment 16 is the method, system, or computer-readable medium of any one of the preceding embodiments, wherein a first fluorescence measurement is obtained from each of a plurality of reaction chambers in step a).

Embodiment 17 is the method, system, or computer-readable medium of the immediately preceding embodiment, wherein step b) comprises introducing, or attempting to introduce, a first liquid into each of the plurality of reaction chambers in step b).

Embodiment 18 is the method, system, or computer-readable medium of the immediately preceding embodiment, wherein step c) comprises obtaining a second fluorescent measurement from each of the plurality of reaction chambers in step c).

Embodiment 19 is the method, system, or computer-readable medium of the immediately preceding embodiment, wherein step d) comprises determining a first value from each of the plurality of first fluorescence measurements and each of the plurality of second fluorescence measurements, each first value indicating a degree of change in fluorescence in the corresponding reaction chamber resulting from step b).

Embodiment 20 is the method, system, or computer-readable medium of the immediately preceding embodiment, wherein step e) comprises comparing each first value to a first predetermined threshold or range, wherein an abnormality is detected if any first value does not satisfy the first predetermined threshold or any first value is outside the first predetermined range.

Embodiment 21 is the method, system, or computer-readable medium of any one of embodiments 16-20, wherein the plurality of reaction chambers is contained in a multi-well plate or in a plurality of tubes.

Embodiment 22 is the method, system, or computer-readable medium of any one of the preceding embodiments, wherein the first liquid comprises a fluorophore.

Embodiment 23 is the method, system, or computer-readable medium of the immediately preceding embodiment, wherein the fluorophore is associated with an oligonucleotide probe, optionally wherein the oligonucleotide probe further comprises a quencher.

Embodiment 24 is the method, system, or computer-readable medium of any one of embodiments 1-21, wherein the first liquid does not comprise a fluorophore associated with an oligonucleotide and inherent fluorescence of the one or more reagents or a sample is measured, optionally wherein the one or more reagents comprise dNTPs and/or one or more primers.

Embodiment 25 is the method, system, or computer-readable medium of any one of the preceding embodiments, wherein the method further comprises, after step c):
i) introducing, or attempting to introduce, a second liquid into the reaction chamber;
ii) after step i), obtaining a third fluorescent measurement from the reaction chamber;
iii) determining a second value from the second fluorescent measurement and the third fluorescent measurement, the second value indicating a degree of change in fluorescence in the reaction chamber resulting in step i); and
iv) comparing the second value to a second predetermined threshold or range, wherein an abnormality is detected if the second value does not satisfy the second predetermined threshold or the second value is outside the second predetermined range.

Embodiment 26 is the method, system, or computer-readable medium of the immediately preceding embodiment, wherein the second liquid comprises one or more nucleic acid amplification reagents or a sample different from the one or more nucleic acid amplification reagents or nucleic acid sample of the first liquid.

Embodiment 27 is the method, system, or computer-readable medium of embodiment 25 or 26, wherein the second value is determined as a ratio of the third fluorescence measurement to the second fluorescence measurement.

Embodiment 28 is the method, system, or computer-readable medium of any one of embodiments 25-27, wherein the third fluorescence measurement is an average or median of a plurality of individual measurements acquired in step ii).

Embodiment 29 is the method, system, or computer-readable medium of any one of embodiments 25-28, wherein after step i) and before step ii), a nucleic acid amplification reaction is initiated.

Embodiment 30 is the method, system, or computer-readable medium of the immediately preceding embodiment, wherein initiation of the nucleic acid amplification reaction comprises initiating a denaturation or reverse transcription step.

Embodiment 31 is the method, system, or computer-readable medium of the immediately preceding embodiment, wherein the third fluorescence measurement is obtained during the denaturation or reverse transcription step.

Embodiment 32 is the method, system, or computer-readable medium of any one of embodiments 25-31, wherein the first liquid and the second liquid together form a nucleic acid amplification reaction mixture.

Embodiment 33 is the method, system, or computer-readable medium of the immediately preceding embodiment, wherein the nucleic acid amplification reaction mixture is a thermocycled nucleic acid amplification reaction mixture.

Embodiment 34 is the method, system, or computer-readable medium of embodiment 32 or 33, wherein the nucleic acid amplification reaction mixture is contained within a microfluidic cartridge.

Embodiment 35 is the method, system, or computer-readable medium of the immediately preceding embodiment, wherein the microfluidic cartridge comprises:
a) a plurality of functional areas comprising a sample preparation area, a nucleic acid amplification area, and a waste area;
b) a central distribution hub; and
c) a pump, a plurality of valves, and a fluidic network of microchannels connecting the functional areas to the hub;
wherein the pump, plurality of valves, and fluidic network of microchannels can drive movement of a fluid from a first functional area through the central distribution hub to a second functional area of the plurality of functional areas.

Embodiment 36 is the method, system, or computer-readable medium of the immediately preceding embodiment, wherein the nucleic acid amplification reaction mixture is located in the nucleic acid amplification area during step ii).

Embodiment 37 is the method of any one of embodiments 25-36, wherein the second liquid is introduced into the reaction chamber and no abnormality is detected in step iii).

Embodiment 38 is the method of any one of embodiments 25-36, wherein the second liquid is not introduced into the reaction chamber and an abnormality is detected in step iii).

Embodiment 39 is the method of any one of embodiments 25-36, wherein step i) comprises attempting to introduce a first volume of the second liquid into the reaction chamber but a second volume of the second liquid is introduced into the reaction chamber, the second volume being less than the first volume, and an abnormality is detected in step iv).

Embodiment 40 is the method of any one of embodiments 25-36, wherein step i) comprises attempting to introduce a first volume of the second liquid into the reaction chamber and the first volume within a tolerance of 10%, 5%, 2%, 1%, or 0.5% is introduced into the reaction chamber, and no abnormality is detected in step iii).

Embodiment 41 is the method, system, or computer-readable medium of any one of the preceding embodiments, wherein the abnormality is defective liquid handling.

Embodiment 42 is the method of any one of embodiments 1 and 4-41, wherein the first liquid is introduced into the reaction chamber and no abnormality is detected in step e).

Embodiment 43 is the method of any one of embodiments 1 and 4-41, wherein the first liquid is not introduced into the reaction chamber and an abnormality is detected in step e).

Embodiment 44 is the method of any one of embodiments 1 and 4-41, wherein step b) comprises attempting to introduce a first volume of the first liquid into the reaction chamber but a second volume of the first liquid is introduced into the reaction chamber, the second volume being less than the first volume, and an abnormality is detected in step e).

Embodiment 45 is the method of any one of embodiments 1 and 4-41, wherein step b) comprises attempting to introduce a first volume of the first liquid into the reaction chamber and the first volume is introduced into the reaction chamber within a tolerance of 10%, 5%, 2%, 1%, or 0.5%, and no abnormality is detected in step e).

Embodiment 46 is the system of any one of embodiments 2, 4-36, and 41, wherein the system is configured not to subject the reaction chamber to nucleic acid amplification conditions if an abnormality is detected and/or is configured to subject the reaction chamber to nucleic acid amplification conditions only if an abnormality is not detected.

Embodiment 47 is the system or computer-readable medium of any one of embodiments 2-36, 41, and 45, wherein the method further comprises subjecting the reaction chamber to nucleic acid amplification conditions only if an abnormality is not detected.

Embodiment 48 is the system or computer-readable medium of embodiment 45 or 46, wherein the nucleic acid amplification conditions comprise thermocycling.

Embodiment 49 is the system or computer-readable medium of any one of embodiments 2-36, 41, and 46-48, wherein the method further comprises discontinuing a nucleic acid amplification reaction if an abnormality is detected.

Additional objects and advantages will be set forth in part in the description which follows, and in part will be understood from the description, or may be learned by practice. The objects and advantages will be realized and attained by means of the elements and combinations particularly pointed out in the appended claims.

It is to be understood that both the foregoing general description and the following detailed description are exemplary and explanatory only and are not restrictive of the claims.

### BRIEF DESCRIPTION OF THE DRAWINGS

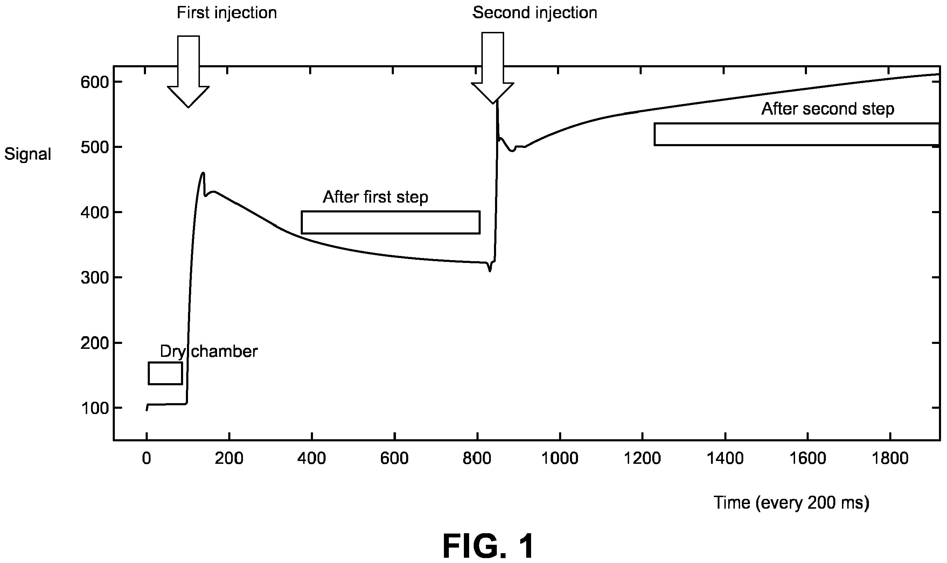

Figure 1 is an exemplary graph of fluorescence over time during monitoring liquid handling of a reaction chamber. Times of first and second attempts to introduce first and second liquids into a reaction chamber are indicated (see arrows). Fluorescence measurements were obtained before the first attempt to introduce liquid, and after each of the first and second attempts to introduce liquid (see horizontal bars). Relative fluorescence units (signal; RFUs; y-axis) are plotted versus time (milliseconds on the x-axis).

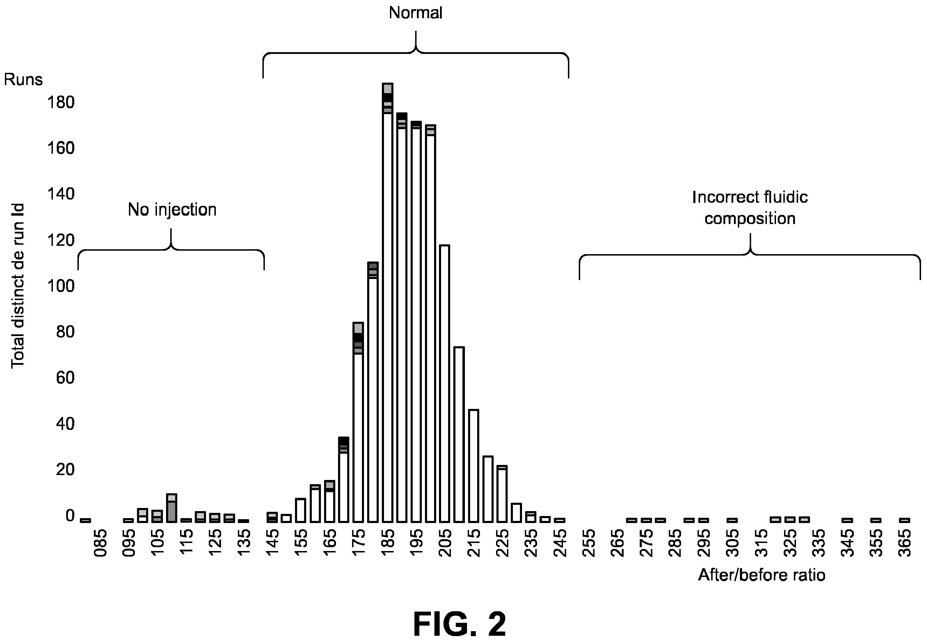

Figure 2 is an exemplary histogram of the ratio of fluorescence before and after an attempt to introduce a liquid into a reaction chamber for a plurality of attempts as described in Example 1. The histogram shows the number of conditions (y-axis) versus the after/before ratios of fluorescence obtained after the second injection (x-axis) for each of those chambers. The ranges of values typically corresponding to chambers with normal liquid handling conditions ("Normal"), no injection, and an incorrect fluidic composition are indicated.

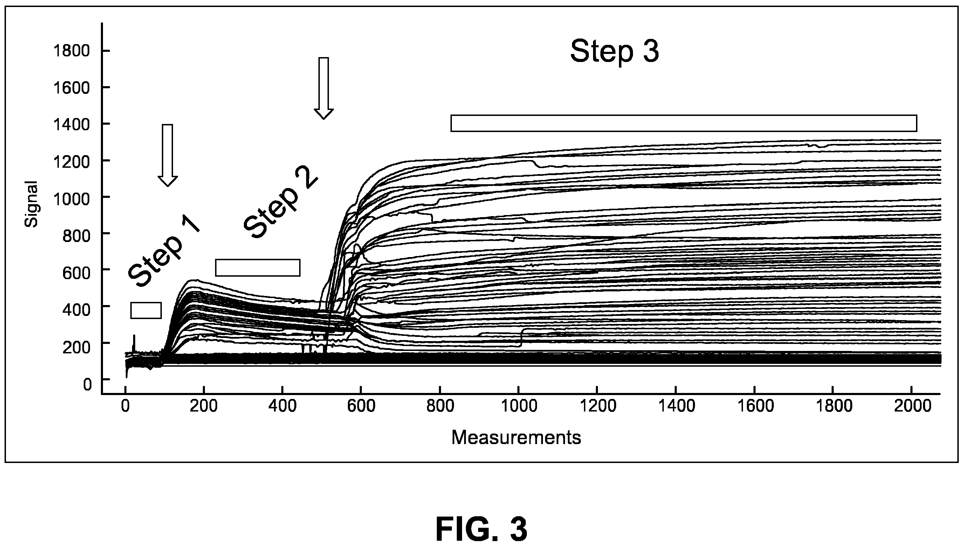

Figure 3 shows exemplary fluorescence signals from a plurality of qPCR amplification reactions with diverse liquid handling abnormalities or no abnormality (e.g., correct liquid filling). Times corresponding to first and second attempts to introduce a first and a second liquid into a reaction chamber are indicated with arrows. Fluorescence measurements are obtained before the first attempt to introduce liquid (during Step 1), and after each of the first and second attempts to introduce liquid (during Steps 2 and 3, respectively). Relative fluorescence units (signal; RFUs; y-axis) are plotted versus measurements (taken once every 200 ms, x-axis).

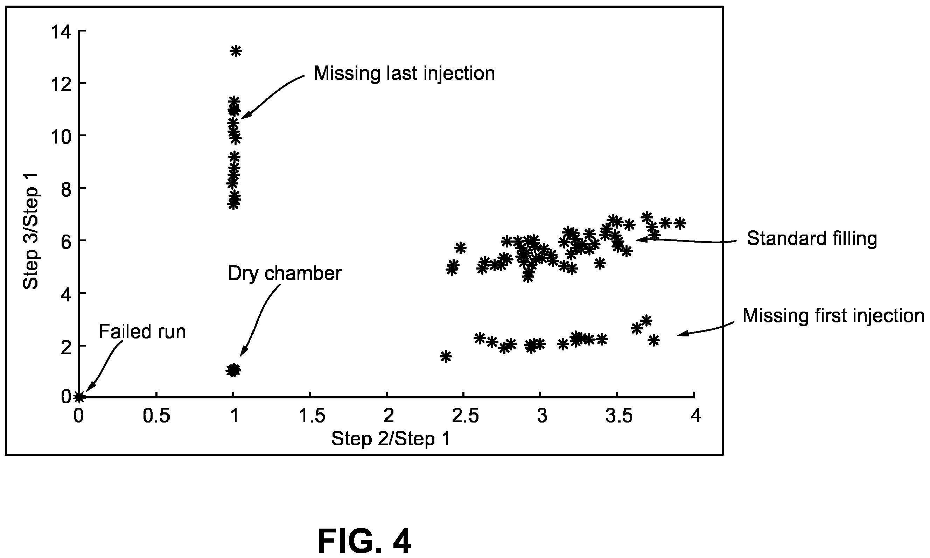

Figure 4 is an exemplary two-dimensional plot of data from reactions with various abnormalities such as missing injection steps or no abnormality, plotted as the ratio of the median fluorescent measurements from Steps 3 and 1 (y-axis) versus the ratio of the median fluorescent measurements from Steps 2 and 1 (x-axis), as shown in Figure 3.

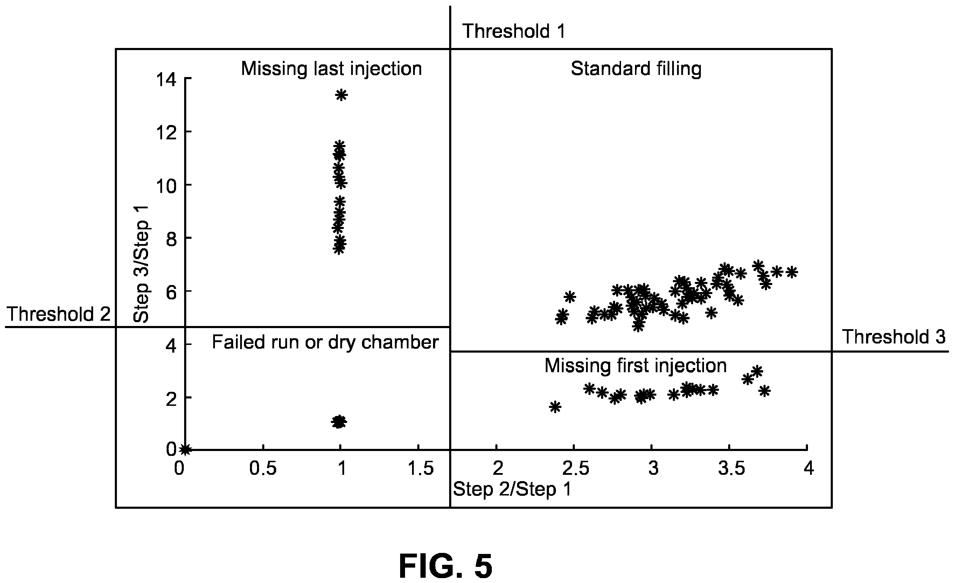

Figure 5 is the 2-dimensional plot depicted in Figure 4, marked with exemplary thresholds (thr1, thr2, and thr3) used to detect and/or identify an abnormality in liquid filling.

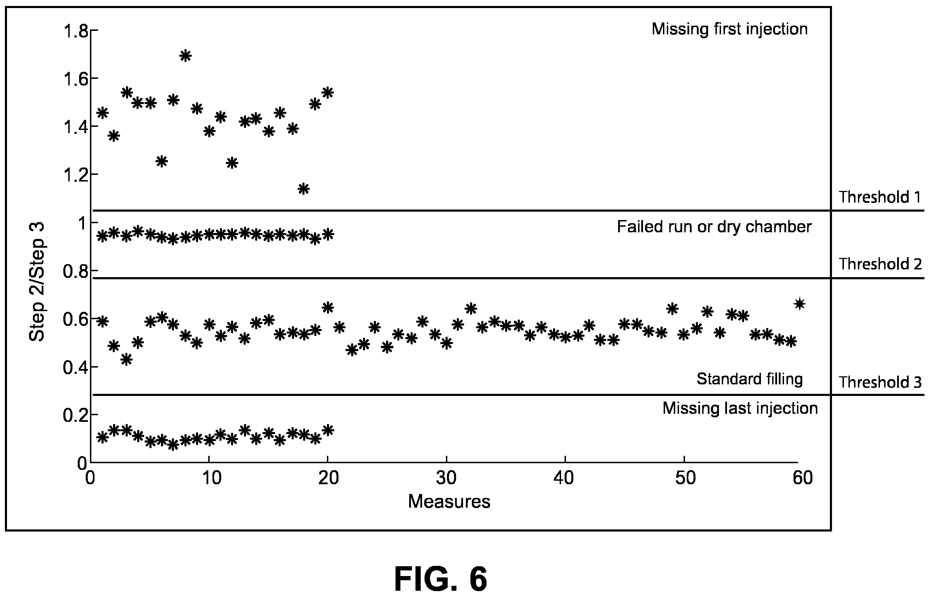

Figure 6 is an exemplary two-dimensional plot of data from reactions with various abnormalities such as missing injection steps, plotted as ratios of the median fluorescent measurements from Steps 2 and 3 (y-axis) versus number of runs (x-axis), as shown in Figure 3. The 2-dimensional map is marked with alternative exemplary thresholds (thr1, thr2, and thr3) used to detect and/or identify an abnormality in liquid filling.

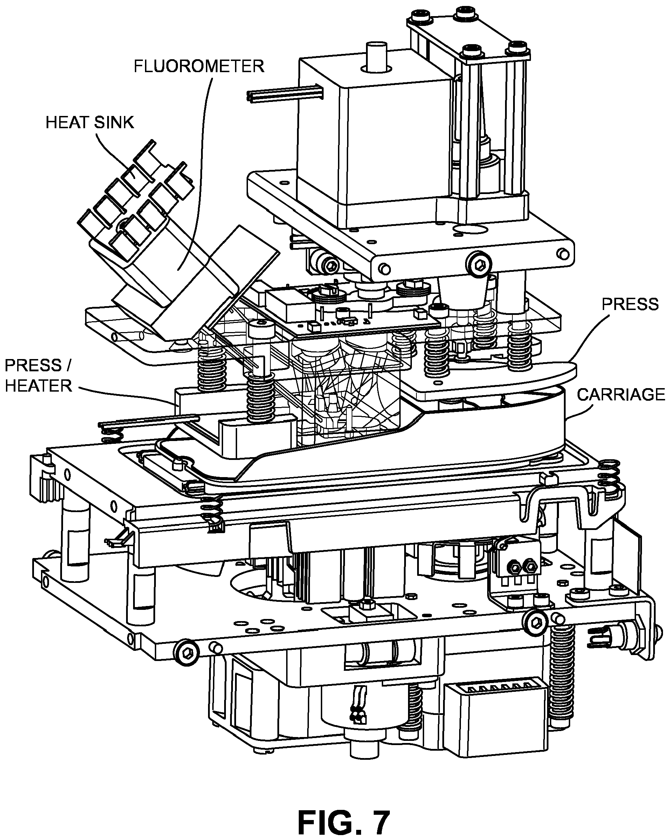

Figure 7 is an exemplary system capable of nucleic acid amplification loaded with a microfluidic cartridge in its docking station. The system includes a heat sink, a fluorometer, and a press/ heater used for interacting and measuring a sample loaded in the cartridge.

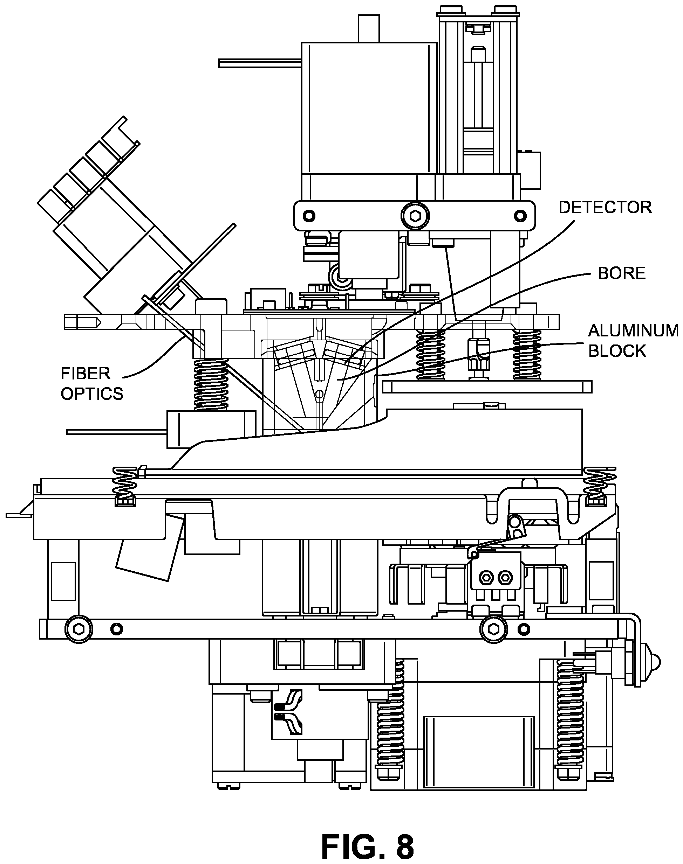

Figure 8 is a side view of the docking station of Figure 7. The side view shows the fiber optics that run from the cartridge to the fluorometer as well as the detector, bore and aluminum block used for taking fluorescence measurements.

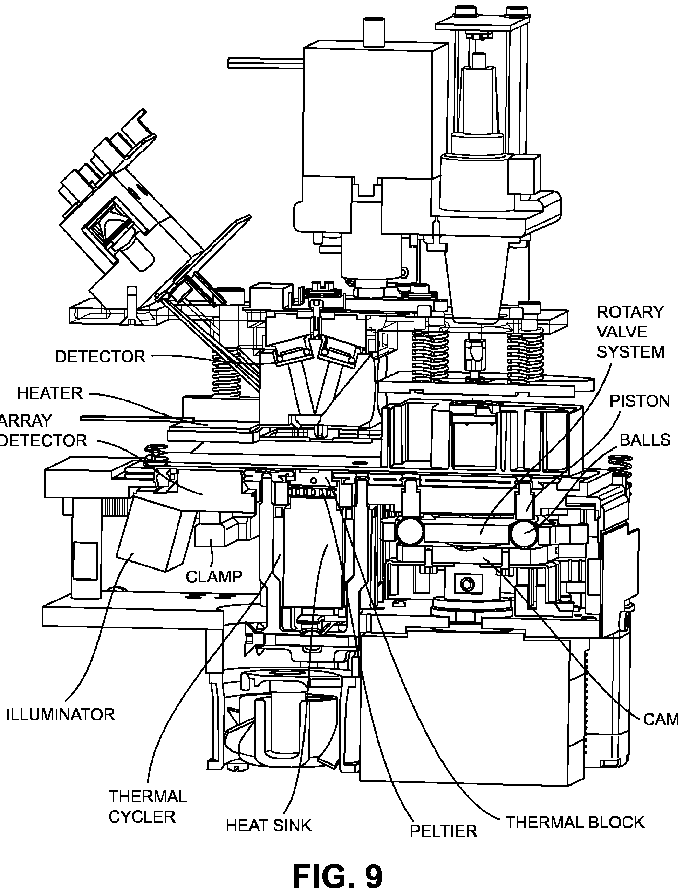

Figure 9 shows a cutaway view of the nucleic acid amplification system docking station and a microfluidic cartridge of Figures 7 and 8. The cutaway view shows internal components such as the thermal cycler, thermal block, array detector, rotary valve system and pistons useful for moving and analyzing liquids in different functional areas of the cartridge.

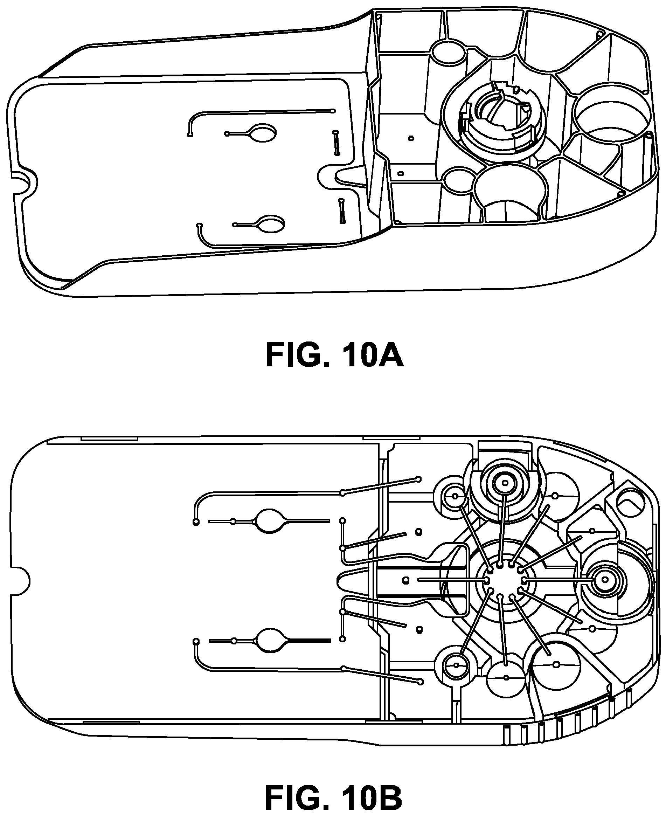

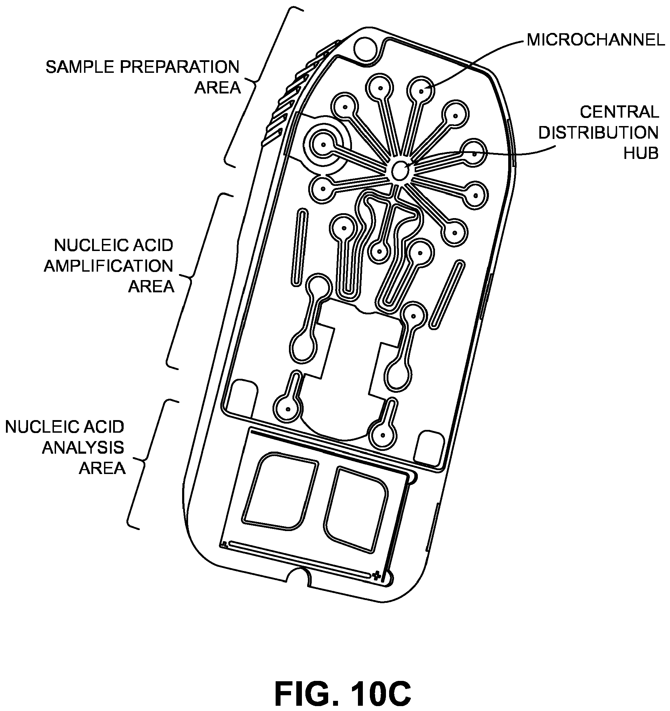

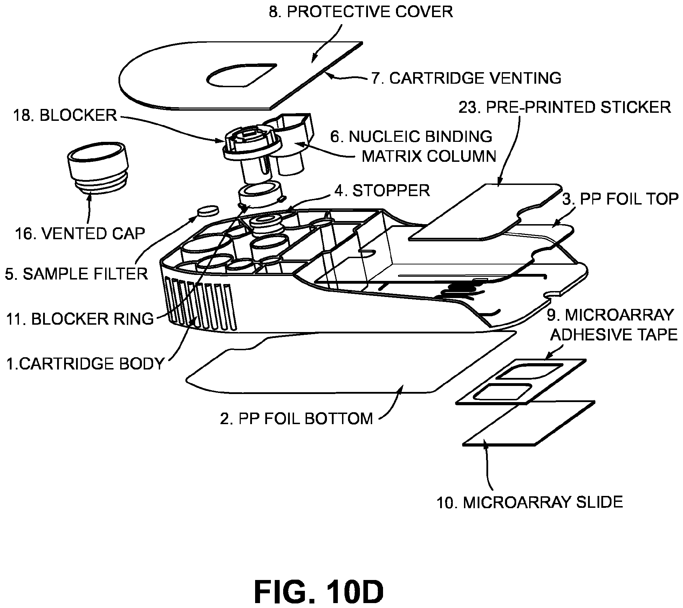

Figures 10A-10D show various views of an exemplary microfluidic cartridge. Figure 10A shows a top-down view of a microfluidic cartridge which contains multiple chambers for housing samples, reagents, or other liquids. Figure 10B shows the bottom view of the exemplary microfluidic cartridge which shows a fluidic network of microchannels connecting various chambers to other areas within the cartridge. Figure 10C shows a bottom view of a fully constructed microfluidic cartridge which includes a sample preparation area, a nucleic acid amplification area, and a nucleic acid analysis area. These functional areas are connected by the series of microchannels shown in detail in Figure 10B. The sample preparation area includes liquids in adjacent chambers. The system can be programmed to combine liquids contained in these chambers in discrete volumes in a particular order. When docked in the system, the nucleic acid amplification area is in close proximity to the thermal cycler in Figure 8. Similarly, when docked in the system, the nucleic acid analysis area is in close proximity to the array detector as shown in Figure 9. Figure 10D shows an exploded view of the fully constructed exemplary microfluidic cartridge which includes a cartridge body (1) a vented cap for covering the sample after input (16), a sample filter (5) and the components for the cartridge to interact with the mechanical components of the system to move and combine liquids in the sample preparations area (4, 6-8, 11). The exploded view also shows how the microarray slide (10) is attached to the nucleic acid amplification area via an adhesive tape (9) in the nucleic amplification analysis area. The cartridge also includes polypropylene (PP) foil coverings (2, 3).

### DEFINITIONS

Unless defined otherwise, all technical and scientific terms used herein have the same meaning as commonly understood by one of ordinary skill in the art pertinent to the methods and compositions described. All patents, applications, published applications and other publications referred to herein are incorporated by reference in their entirety. If a definition set forth in this section is contrary to or otherwise inconsistent with a definition set forth in the patents, applications, published applications, and other publications that are herein incorporated by reference, the definition set forth in this section prevails over the definition that is incorporated herein by reference.

"Liquid handling" refers to the aspirating, movement, and dispensing of liquids. "Liquid filling" refers to the filling of reaction chambers with liquids via liquid handling. Manual or automated methods of liquid handling may be employed. Abnormalities in the liquid filling of chambers may arise from errors in liquid handling.

"Defective liquid handling" refers to the aspirating, movement, and dispensing of liquids that results in a deviation from the intended volume or composition in the reaction chamber following an attempt to introduce a liquid into the reaction chamber.

"Nucleic acid" and "polynucleotide" refers to a multimeric compound including two or more covalently bonded nucleosides or nucleoside analogs having nitrogenous heterocyclic bases, or base analogs, where the nucleosides are linked together by phosphodiester bonds or other linkages to form a polynucleotide. Nucleic acids include RNA, DNA, and combinations and analogs thereof such as "peptide nucleic acids" or PNAs (see, e.g., WO 95/32305) and "locked nucleic acids" (LNA), in which one or more nucleotide monomers have a bicyclic furanose unit locked in an RNA mimicking sugar conformation (see, e.g., Vester et al., Biochemistry 43:13233-41, 2004). Nitrogenous bases may be conventional bases (A, G, C, T, U), analogs thereof (e.g., inosine, 5-methylisocytosine, isoguanine; see, e.g., The Biochemistry of the Nucleic Acids 5-36, Adams et al., ed., 11th ed., 1992; Abraham et al., 2007, BioTechniques 43: 617-24), which include derivatives of purine or pyrimidine bases (e.g., N⁴-methyl 12deoxyguanosine, deaza- or aza-purines, deaza- or aza-pyrimidines, etc.; U.S. Pat. Nos. 5,378,825, 6,949,367 and International Patent Application Pub. No. WO 93/13121), and/or "abasic" residues (see. e.g., U.S. Pat. No. 5,585,481).

A "target" material as used herein, is a material to be detected or quantified. A , target material may be a nucleic acid; other embodiments of target materials include cells, viruses, and other biomolecules. A "target nucleic acid" as used herein is a nucleic acid including a target sequence to be detected or quantified, e.g., via amplification. Target nucleic acids or target nucleic acids of interest may be DNA or RNA or combinations or analogs thereof as described herein and may be either single-stranded or double-stranded. The target nucleic acid may include other sequences besides the target sequence, which may not be detected or quantified.

"Primer," "amplification oligonucleotide," or "oligonucleotide primer" refers to a polynucleotide, generally an oligonucleotide including a "target" binding portion, that is designed to selectively hybridize with a target nucleic acid flanking sequence or to a corresponding primer-binding site of an amplification product under appropriate stringency conditions and serve as the initiation point for the synthesis of a nucleotide sequence that is complementary to the corresponding polynucleotide template from its 3'-end. The 5' region of the primer may be non-complementary to the target nucleic acid. If the 5' non-complementary region includes a promoter sequence, it is referred to as a "promoter-primer."

The term "region," as used herein, refers to a portion of a nucleic acid wherein said portion may be smaller than the entire nucleic acid. For example, the term "region" may be used to refer to a smaller target-hybridizing portion of the entire oligonucleotide. Certain oligonucleotides, such as primers, may consist entirely of one region (e.g., target-hybridizing region) or may contain a plurality of regions (e.g., a promoter sequence region and a target-hybridizing region, in a promoter-primer).

"Sample" refers to any composition that may contain or is suspected of containing a target material. A sample may be a complex mixture of components. Samples include "biological samples" which include any tissue or material derived from a living or dead mammal or organism, including, for example, stool, blood, plasma, serum, blood cells, saliva, mucous and cerebrospinal fluid. Samples include "nucleic acid samples" which include nucleic acids, as described above. Samples may also include samples of in vitro cell culture constituents including, for example, conditioned media resulting from the growth of cells and tissues in culture medium. Samples also include food, which includes any material intended or suitable for consumption, including solids, suspensions, emulsions, gels, and liquids (thus including gelatin, milk, soups, beverages, ice-cream, fruit smoothies, emulsified cheese dips, fruit puree, nut butter, processed and/or textured protein, and the like, as well as bread, fruit, vegetables, and meat.). Samples also include water and aqueous solutions. A sample may be treated chemically, physically, or mechanically to disrupt tissue or cell structure to release intracellular nucleic acids into a solution. Samples may be treated to release nucleic acids into a solution containing enzymes, buffers, salts, detergents and the like.

The interchangeable terms "oligomer," "oligo," and "oligonucleotide" refer to a nucleic acid having generally less than 1,000 nucleotide (nt) residues, including polymers in a range having a lower limit of about 5 nt residues and an upper limit of about 500 to 900 nt residues. In some embodiments, oligonucleotides are in a size range having a lower limit of about 12 to 15 nt and an upper limit of about 50 to 600 nt, and other embodiments are in a range having a lower limit of about 15 to 20 nt and an upper limit of about 22 to 100 nt. An oligonucleotide may serve one or more of various different functions, e.g., as a primer and/or promoter, detection probe, capture oligomer, etc.

"Amplifying" or "nucleic acid amplification" refers to any known procedure for obtaining multiple copies of a target nucleic acid sequence or its complement or fragments thereof. The multiple copies may be referred to as amplicons or amplification products. As used herein, the term "nucleic acid amplification conditions" refers to both temperature and chemical conditions that enable nucleic acid amplification. Methods of forming a reaction mixture, and subjecting the reaction mixture to conditions suitable for nucleic acid amplification are well established. Known amplification methods include both thermal cycling and isothermal amplification methods. Polymerase chain reaction (PCR), replicase-mediated amplification, ligase chain reaction (LCR), strand-displacement amplification (SDA), and transcription-associated amplification (e.g., transcription-mediated amplification (TMA) or NASBA) are non-limiting examples of nucleic acid amplification methods. See, e.g., US Pat. Nos. 4,868,105; 5,124,246; 5,130,238; 5,399,491; 5,437,990; 5,554,516; and 7,374,885; and PCT Pub. Nos. WO 88/01302; WO 88/10315 and WO 95/03430 (TMA); US Pat. No. 4,786,600 (RCA); US Pat. No. 5,427,930 and US Pat. No. 5,516,663 (LCR); and US Pat. No. 5,422,252; US Pat. No. 5,547,861; and US 5,648,211 (SDA). See also, e.g., Compton, Nature 350:91-92, 1991; Malek et al., Methods Mol. Biol. 28:253-260, 1994 (NASBA). Briefly, PCR amplification uses a DNA polymerase, pairs of primers, and thermal cycling to synthesize multiple copies of two complementary strands from dsDNA or from a cDNA (see, e.g., US Pat. Nos. 4,683,195, 4,683,202, and 4,800,159).

As used herein, the term "real-time amplification" refers to amplification of target nucleic acid that is monitored by real-time detection. Real-time PCR amplification includes, e.g., what is commonly referred to as Taqman^{®} PCR (see, e.g., Holland et al., Proc. Natl. Acad. Sci. USA 88:7276-7280, 1991; and Livak et al., US Pat. No. 6,030,787). Taqman PCR is a type of real-time PCR that uses a nucleic acid probe complementary to an internal segment of the target DNA. The probe is labeled with two fluorescent moieties. The emission spectrum of one overlaps the excitation spectrum of the other, resulting in "quenching" of the first fluorophore by the second.

As used herein, "thermocycling" is a process of cyclically heating and cooling of a nucleic acid amplification mixture that facilitates amplifying nucleic acids through successive rounds of denaturation, primer annealing, and primer extension, e.g., by a thermostable polymerase. In many examples, thermocycling includes holding a reaction mixture at two or more different temperatures, each for a predetermined length of time, and performing several cycles of those two or more temperatures to cause nucleic acid amplification. A nucleic acid amplification reaction mixture that has been subjected to thermocycling is termed a "thermocycled reaction mixture."

As used herein, a "nucleic acid amplification system" refers to a device or apparatus that may be used to perform nucleic acid amplification. In many examples, the nucleic acid amplification system includes a fluorometer and is capable of obtaining fluorescence measurements prior to, during and after nucleic acid amplification. In many examples, the nucleic acid amplification system includes a temperature controller and is capable of providing or transferring heat to nucleic acid amplification reaction mixtures via one or more heating elements, which may include, e.g., a heat block. The nucleic acid amplification system is typically programmable and can hold temperatures for different lengths of time. The nucleic acid amplification system may be configured to accommodate any one or more of a variety of reaction vessels, such as tubes, multi-well strips, multi-well plates, microfluidic chips, and microfluidic cartridges, which may contain one or more nucleic acid amplification mixtures.

The term "amplicon" or the term "amplification product" as used herein refers to the nucleic acid molecule generated during an amplification procedure that is complementary or homologous to a sequence contained within the target sequence. These terms can be used to refer to a single-stranded amplification product, a double-stranded amplification product, or one of the strands of a double-stranded amplification product.

By "complementary" is meant that the nucleotide sequences of similar regions of two single-stranded nucleic acids, or two different regions of the same single-stranded nucleic acid, have nucleotide base compositions that allow the single-stranded regions to hybridize together in a stable double-stranded hydrogen-bonded region under stringent hybridization or amplification conditions. Hybridization or amplification conditions are "stringent" enable the hybridization of highly homologous nucleic acid sequences. Such conditions may include high hybridization temperatures and a low concentration of salt in the buffers. Sequences that hybridize to each other may be completely complementary or partially complementary to the intended target sequence by standard nucleic acid base pairing (e.g., G:C, A:T, or A:U pairing). By "sufficiently complementary" is meant a contiguous sequence that is capable of hybridizing to another sequence by hydrogen bonding between a series of complementary bases, which may be complementary at each position in the sequence by standard base pairing or may contain one or more residues, including abasic residues, that are not complementary. Sufficiently complementary contiguous sequences typically are at least 80%, or at least 90%, complementary to a sequence to which an oligomer is intended to specifically hybridize. Sequences that are "sufficiently complementary" allow stable hybridization of a nucleic acid oligomer with its target sequence under appropriate hybridization conditions, even if the sequences are not completely complementary. When a contiguous sequence of nucleotides of one single-stranded region is able to form a series of "canonical" or "Watson-Crick" hydrogen-bonded base pairs with an analogous sequence of nucleotides of the other single-stranded region, such that A is paired with U or T and C is paired with G, the nucleotides sequences are "completely" complementary (see. E.g., Sambrook et al., Molecular Cloning: A Laboratory Manual, 2nd ed. (Cold Spring Harbor Laboratory Press, Cold Spring Harbor, N. Y., 1989) at §§1.90-1.91, 7.37-7.57, 9.47-9.51 and 11.47-11.57, particularly §§9.50- 9.51, 11.12-11.13, 11.45-11.47 and 11.55-11.57). Appropriate hybridization conditions are well-known in the art, may be predicted based on sequence composition, or can be determined by using routine testing methods (see e.g., Sambrook et al., supra.)

As used herein, a "label" or "detection label" refers to a moiety or compound that can be detected or generate a detectable signal and that is joined directly or indirectly to a molecule, such as a probe. Direct labeling can occur through bonds or interactions that link the label to the molecule, including covalent bonds or non-covalent interactions, e.g., hydrogen bonds, hydrophobic and ionic interactions, or formation of chelates or coordination complexes. Indirect labeling can occur through use of a bridging moiety or "linker" such as a binding pair member, an antibody or additional oligomer, which is either directly or indirectly labeled, and which may amplify the detectable signal. Labels include any detectable moiety, such as a radionuclide, ligand (e.g., biotin, avidin), enzyme or enzyme substrate, reactive group, or chromophore (e.g., dye, particle, or bead that imparts detectable color), luminescent compound (e.g., bioluminescent, phosphorescent, or chemiluminescent labels), or fluorophore. Common labels used for TaqMan^{®} probes include a fluorophore and a quencher. "Fluorophore" as used herein refers to any label whose presence can be detected by its fluorescent light emitting properties. The term "quencher" as used herein refers to a moiety that absorbs at least some of the intensity of a fluorescent emission. Quenchers include fluorescent quenchers and dark quenchers (sometimes also referred to as non-fluorescent quenchers). A dark quencher is a substance that absorbs excitation energy from a fluorophore and dissipates the energy as heat, while a fluorescent quencher re-emits much of this energy as light. A fluorescent quencher is a moiety, typically a fluorophore, that can absorb the fluorescent signal emitted from a source of fluorescence at a first wavelength, for example but not limited to, a nucleic acid dye associated with a double-stranded segment of nucleic acid, and after absorbing enough fluorescent energy, the fluorescent quencher can emit fluorescence at a second wavelength that is characteristic of the quencher, a process termed "fluorescent resonance energy transfer" or FRET. Exemplary fluorophores include FAM, SYBR^{®} Green, ATTO fluorescent labels, VIC, JOE, NED, Cy3, ROX, Texas Red and Cy5 dyes (all available from numerous commercial sources). Exemplary quenchers include BBQ, ATTO quenchers, BHQ, TAMRA and DABCYL (all available from numerous commercial sources). Synthesis and methods of attaching labels to nucleic acids and detecting labels are known in the art (see for example, Sambrook et al., Molecular Cloning, A Laboratory Manual, 2nd ed. (Cold Spring Harbor Laboratory Press, Cold Spring Harbor, NY, 1989), Chapter 10; US Pat. Nos. 5,658,737, 5,656,207, 5,547,842, 5,283,174, and 4,581,333). More than one label, and more than one type of label, may be present on a particular probe, or detection may use a mixture of probes in which each probe is labelled with a compound that produces a different detectable signal (see, e.g., US Pat. Nos. 6,180,340 and 6,350,579).

"Detection probe," "detection oligonucleotide," "detection oligomer," "probe oligomer," and "detection probe oligomer" are used interchangeably to refer to a nucleic acid oligomer that hybridizes specifically to a target sequence in a nucleic acid, such as an amplified nucleic acid, under conditions that promote hybridization to allow detection of the target sequence or amplified nucleic acid. Detection may either be direct (e.g., a probe hybridized directly to its target sequence) or indirect (e.g., a probe linked to its target via an intermediate molecular structure). Detection probes may be DNA, RNA, analogs thereof, or combinations thereof (e.g., DNA/RNA chimerics) and they may be labeled or unlabeled; Detection probes may further include alternative backbone linkages such as, e.g., 2'-O-methyl linkages. A detection probe's "target sequence" generally refers to a smaller nucleic acid sequence region within a larger nucleic acid sequence that hybridizes specifically to at least a portion of a probe oligomer by standard base pairing. A detection probe may include target-specific sequences and other sequences that contribute to the three-dimensional conformation of the probe (see, e.g., U.S. Pat. Nos. 5,118,801; 5,312,728; 6,849,412; 6,835,542; 6,534,274; and 6,361,945; and US Patent Application Pub. No. 20060068417).

An "elution buffer" as used herein is a suitable liquid for separating nucleic acids from a solid support.

A "master mix buffer," "master mix," or "amplification master mix buffer" as used herein includes amplification reagents and optionally primers used to amplify a target nucleic acid but does not include the sample to be amplified.

A "reaction chamber," also referred to as a "reaction space" denotes a space in which the amplification of target nucleic acids is performed. Reaction chambers include tubes or wells. Multiple reaction chambers may be arranged in parallel or substantially parallel to each other in a variety of vessels. Examples of vessels that can contain multiple reaction chambers include a multi-well strip, a multi-well plate, a microfluidic chip, or a microfluidic cartridge.

A "fluorometer," "fluorescence detector," or "fluorescence sensor" as used herein, refers to an optical detector that is capable of obtaining fluorescence measurements. Such measurements can be obtained prior to, during and after nucleic acid amplification. A fluorometer may be a photodiode or photomultiplier. The fluorometer receives light (fluorescence) emitted from a sample. Detection of PCR products in real-time can be accomplished by using fluorescent dyes or probes. The fluorescence signal, as measured by a fluorometer increases at each PCR cycle as more polynucleotide molecules are generated.

As used herein, the term "relative fluorescence unit" ("RFU") is a unit of measurement of fluorescence intensity. RFU varies with the characteristics of the detection equipment used for the measurement and can be used as a measurement to compare relative intensities between samples and controls.

As used herein, the term "substantially" can be synonymous with the term "essentially" and indicates that a step, reagent, component, or other element achieves a result or has a property that does not materially differ, but may have minor differences or deviations, from the result or property to which "substantially" applies. For example, to "substantially avoid variation" may mean that the variation is less than or equal to about 20%, 15%, 10%, 5%, 4%, 3%, 2%, or 1%.

As used herein, the term "about" refers to a numerical value, including, for example, whole numbers, fractions, and percentages, whether or not explicitly indicated. When the term precedes a list of numerical values or ranges, the terms modify all of the values or ranges. As applied to measured quantities, the term includes the exact numerical value modified by the term, as well as ranges of values that would be expected to be within experimental error. For example, "about 5°C" means "5°C" and a range of temperatures that is within experimental error, e.g., plus or minus (±) 20% of 5°C, ±15% of 5°C, ±10% of 5°C, or ±5% of 5°C. The term has a similar meaning with respect to other parameters. For example, "about 5 minutes" means "5 minutes" and a range of times that is within experimental error, e.g., plus or minus (±) 20% of 5 minutes, ±15% of 5 minutes, ±10% of 5 minutes, or ±5% of 5 minutes. In some contexts, the percentage of experimental error is implied or apparent and may not be explicitly stated. In some contexts, the percentage of experimental error will be provided explicitly. The term "about" may be used to modify any measurable quantity, including quantities of time, temperature, volume, mass, weight, length, density, size, percentage, ratio, dose, frequency, pressure, rate, and intensity. In some instances, the term about may include numerical values that are rounded to the nearest significant figure.

Reference to a numerical range herein (e.g., "X to Y" or "from X to Y" or "between X and Y") includes the endpoints defining the range and all values falling within the range.

The terms "a," "an," and "the" include plural referents, unless the context clearly indicates otherwise. For example, "a nucleic acid" as used herein is understood to represent one or more nucleic acids. As such, the terms "a" (or "an"), "one or more," and "at least one" can be used interchangeably herein.

"Or" is used in the inclusive sense, i.e., is equivalent to "and/or," unless the context clearly indicates otherwise.

### DETAILED DESCRIPTION

Disclosed herein are methods for monitoring the introduction of one or more liquids into reaction chambers for detecting abnormalities in the reaction chambers. The methods can leverage the fluorescence of labeled reagents such as probes and/or reagents with inherent fluorescence, e.g., dNTPs and primers, to detect changes in the volume and/or composition of liquid in the reaction chamber and thereby facilitate detection of abnormalities such as a failed attempt to introduce liquid into the chamber or incorrect liquid filling. Typically, an abnormal liquid filling event results in an error in the final volume and/or composition of a nucleic acid amplification reaction mixture, which can cause failure of the nucleic acid amplification reaction. The methods can thus enable detection of abnormalities in a reaction chamber that may adversely affect the nucleic acid amplification reaction. Importantly, the methods can be implemented on many existing nucleic acid amplification systems, such as real-time nucleic acid amplification systems and any other systems equipped with a fluorometer, without requiring additional hardware. Instead, the methods use the existing features of such systems to monitor fluorescence from a reaction chamber before and after one or more attempts to introduce a liquid into the reaction chamber and thereby detect whether there was an abnormality in liquid filling.

### A. Detection Labels and Probes

Detection labels (e.g., fluorophores) may be used in accordance with the present disclosure. First and/or second liquids and nucleic acid amplification reaction mixtures of the present disclosure may include detection oligomers (e.g., designed to hybridize to an amplicon) and/or amplification oligomers, such as forward and/or reverse primers, with detection labels. In general, an amplification oligomer or detection oligomer with a detection label used in an amplification reaction includes at least (1) a region for hybridizing specifically to a region on a target nucleic acid sequence and (2) a detection label. In some embodiments, an oligomer with a detection label is termed a detection probe.

Suitable fluorophores for use as detection labels can include compounds that emit a detectable light signal, e.g., fluorophores ("fluorescent dye compounds"). More than one label, and more than one type of label, may be present on a particular probe, or a mixture of probes may be used in which each probe is labeled with a compound that produces a detectable signal (see, e.g., US Pat. Nos. 6,180,340 and 6,350,579). Labels may be attached to a probe by various means including covalent linkages, chelation, and ionic interactions, but preferably the label is covalently attached. Suitable fluorophores are well-known in the art and include, for example, CAL Flour^{®} Orange 560, CAL Flour^{®} Red 610, FAM, or ATTO 490LS. In some embodiments, the fluorophore is a temperature-sensitive fluorophore. In some embodiments, the temperature-sensitive fluorophore is sulforhodamine. In embodiments including fluorophore-labeled detection probes, each detection probe further includes a quencher. Suitable quenchers are well-known in the art and include, for example, BHQ, TAMRA, and DABCLY. In other embodiments, a detection probe includes both a fluorescent label and a quencher, a combination that is particularly useful in fluorescence resonance energy transfer (FRET) assays. Specific variations of such detection probes include, e.g., a TaqMan^{®} detection probe (Roche Molecular Diagnostics), a "molecular beacon" (see, e.g., Tyagi et al., Nature Biotechnol. 16:49-53, 1998; US Patent Nos. 5,118,801 and 5,312,728), and a "molecular torch" (see, e.g., US Patent Nos. 6,849,412; 6,835,542; 6,534,274; and 6,361,945). In some embodiments, the fluorophore in a nucleic acid amplification reaction mixture is associated with a probe that further includes a quencher, such as a TaqMan^{®} probe.

### B. Reaction Mixtures

In some embodiments, a reaction mixture used in methods or systems described herein or a first or second liquid introduced into a reaction chamber includes one or more amplification oligomers for amplification of a target nucleic acid. In some embodiments, the amplification oligomers include detection labels. The reaction mixture will typically include, and the first or second liquid may include, other reagents suitable for performing *in vitro* amplification such as buffers, salt solutions, appropriate nucleotide triphosphates (e.g., dATP, dCTP, dGTP, dTTP, ATP, CTP, GTP and UTP), and/or enzyme(s) (e.g., DNA polymerase, reverse transcriptase, and RNA polymerase), and may include test sample components, in which an internal control (IC) target nucleic acid may be present. In some embodiments, the reaction mixture is a nucleic acid amplification reaction mixture.

A variety of liquids can be used in the nucleic acid amplification reaction mixtures used according to the present disclosure. In some embodiments, a first or second liquid may contain one or more reagents used in a nucleic acid amplification reaction. Such reagents can include enzymes, master mixes, and other PCR components. In some embodiments, a first or second liquid may contain a sample, e.g., a nucleic acid sample. In some embodiments, a first liquid may contain a sample and a second liquid may contain all or substantially all of the reagents required for a nucleic acid amplification reaction. In some embodiments, a second liquid may contain a sample and a first liquid may contain all or substantially all of the reagents required for a nucleic acid amplification reaction.

### C. Reaction Chambers; Samples

A variety of reaction vessels may be used to receive first and/or second liquids and/or contain the reaction mixtures used according to the present disclosure. In some embodiments, the first or second liquid contains a sample, such as a nucleic acid sample. In some embodiments, the reaction mixture contains a sample, such as a nucleic acid sample. A reaction vessel may include a reaction chamber or a plurality of reaction chambers. In some embodiments, the steps of the methods disclosed herein are performed with reaction chambers that are empty, or with reaction chambers containing nucleic acid amplification reaction mixtures. In some embodiments, a plurality of fluorophore-containing nucleic acid amplification reaction mixtures is contained in a plurality of wells or a plurality of tubes.

Reaction chambers may be configured into a variety of reaction vessels with each reaction vessel including a plurality of reaction chambers. In some embodiments, a reaction vessel includes a plurality of reaction chambers that are empty and/or reaction chambers that contain nucleic acid amplification reaction mixtures. In some embodiments, the vessel is a multi-chambered receptacle. In some embodiments, the nucleic acid amplification chamber is contained within a multi-chambered receptacle. Non-limiting examples of vessels include multi-well strips, multi-well plates, microfluidic chips, and microfluidic cartridges. In some embodiments, a plurality of nucleic acid amplification reaction mixtures is contained in a multi-well plate or in a plurality of tubes.

In some embodiments, at least one nucleic acid amplification reaction mixture is contained in a multi-well strip, a multi-well plate, a microfluidic chip, or a microfluidic cartridge. In some embodiments, the reaction vessel is a microfluidic cartridge. In some embodiments the microfluidic cartridge, also referred to as a "lab-on-a-chip," can perform a complete nucleic acid analysis of a sample, from sample collection to nucleic acid amplification, to the reading of the result(s). Exemplary microfluidic cartridges that can be used to perform the steps of the methods disclosed herein are depicted in Figures 10A-D. Figure 10A shows a top-down view of an exemplary microfluidic cartridge which contains multiple chambers for housing samples, reagents, or other liquids. Microchannels connect such chambers to move liquids among different functional areas within the cartridge. Figure 10B shows the bottom view of the exemplary microfluidic cartridge which shows a network of microchannels connecting various chambers to other areas within the cartridge. Figure 10C shows a bottom view of a fully constructed microfluidic cartridge which includes a sample preparation area, a nucleic acid amplification area, and a nucleic acid analysis area. These functional areas are connected by the series of microchannels shown in detail in Figure 10B. The sample preparation area includes liquids in adjacent chambers. A system, e.g., a nucleic acid amplification system, can be programmed to combine liquids contained in these chambers in discrete volumes in a particular order. When docked in the system, the nucleic acid amplification area is in close proximity to a thermal cycler, as shown in Figure 8. Similarly, when docked in a system, a nucleic acid analysis area is in close proximity to an array detector as shown in Figure 9. Figure 10D shows an exploded view of the fully constructed exemplary microfluidic cartridge which includes a cartridge body (1) a vented cap for covering the sample after input (16), a sample filter (5) and the components for the cartridge to interact with the mechanical components of the system to move and combine liquids in the sample preparations area (4, 6-8, 11). The exploded view of Figure 10D also shows how the microarray slide (10) is attached to the nucleic acid amplification area via an adhesive tape (9) in the nucleic amplification analysis area. The cartridge also includes polypropylene (PP) foil coverings (2, 3). Microfluidic cartridges have several advantages, including the capability to conduct automated operations while consuming low reagent volumes, while being inexpensive and disposable. Examples of microfluidic cartridges are also disclosed in US Patent No. 10,654,039.

In some embodiments, a microfluidic cartridge includes at least (1) a plurality of functional areas including a sample preparation area, a nucleic acid amplification area, a nucleic acid analysis area and a waste area; (2) a central distribution hub; and (3) a pump, a plurality of valves, and a fluidic network of microchannels connecting the functional areas to the central distribution hub. The pump, plurality of valves, and fluidic network of microchannels can drive movement of a fluid from a first functional area through the central distribution hub to a second functional area of the plurality of functional areas.

The sample preparation area may include of a network of reservoirs or tanks holding a plurality of liquids or gases in various configurations, as shown in Figure 10A. Such liquids or gases may contain a sample and/or reagent commonly used in the art, e.g., master mix, wash buffer, elution buffers hybridization buffers. Arrangement, distribution, and transfer of such reagents may be customized based on experimental protocols or amplification systems used.

The nucleic acid amplification area may be adjacent to the sample preparation area and connected via microfluidic channels. Such channels allow for the movement of prepared samples to the nucleic acid amplification area through the central distribution hub to be subjected to nucleic acid amplification conditions.

The nucleic acid analysis area may include a microarray component or slide, as shown in Figure 10C. A microarray slide may be used to analyze the amplified nucleic acids via capture probes. Such microarrays may include an array of many individual fragments of DNA immobilized on a solid support (e.g., glass slide) that hybridize with complementary target sequences in an organism of interest. Hybridization may be detected using a fluorescent reporter molecule, e.g., a fluorophore. The inclusion of different probe sequences on one microarray allows simultaneous detection of different organisms, or differences between organisms of the same species, allowing for syndromic testing with a high degree of specificity.

In some embodiments where the nucleic acid amplification reaction mixture is contained in a microfluidic cartridge, the nucleic acid amplification reaction mixture may or may not be located in the nucleic acid amplification area. In some embodiments, the first liquid may or may not be located in the nucleic acid amplification area. In these embodiments, if the first liquid was successfully introduced when attempting to introduce a first liquid into the reaction chamber, the first liquid is located in the nucleic acid amplification area when obtaining a second fluorescence measurement from the reaction chamber.

In some embodiments, the second liquid may or may not be located in the nucleic acid amplification area. In some embodiments, the nucleic acid amplification reaction mixture is located in the nucleic acid amplification area when obtaining a third fluorescence measurement from the reaction chamber. In these embodiments, if the second liquid was successfully introduced after attempting to introduce a second liquid into the reaction chamber, the second liquid is located in the nucleic acid amplification area when obtaining a third fluorescence measurement from the reaction chamber.

A functional area may be a space dedicated to a specific operation on the sample. The functional areas of a microfluidic cartridge may be fluidly connected to a central distribution hub by a fluidic network of microchannels. Functional areas can be arranged in a variety of ways, and multiple functional areas can be either identical to or different from each other. Examples of functional areas include a nucleic acid extraction area, a nucleic acid purification area, a nucleic acid preparation area, a nucleic acid hybridization area, a nucleic acid amplification area, a nucleic acid detection area, a nucleic acid analysis area, and a waste area. In some embodiments, the detection area is a biochip.

The central distribution hub can be connected to a pump and a plurality of valves, and the central distribution hub can pump and inject fluids from one functional area to another. Thus, the central distribution hub makes it possible to use only one simple fluid displacement system (typically a pumping system) for most of the fluid movements of the microfluidic cartridge, for inducing depressurization and pressurization in order to displace the fluid from a functional area to another one, and to reduce the volume of the microfluidic cartridge.

Each microchannel may include a central distribution hub end ("the hub end") and a functional area end ("the area end"). The area end of a microchannel is adjacent to the corresponding functional area and, the hub end is adjacent to the central distribution hub. Each microchannel may also include a valve located near the associated area end. Thus, the microfluidic cartridge includes a plurality of valves located at or near the area ends of the hub-connected microchannels.

The valves may be spatially arranged in order to be independently actuated by an actuator. In some embodiments, the actuator is an external cam-driven actuator, linear-motion actuator, a rotational-motion actuator, a linear actuator, or a rotary actuator.

As used herein, a nucleic acid amplification reaction mixture may include a sample, which may include or be suspected of containing a target nucleic acid of interest. A variety of sample types and preparations may be used. In some embodiments, before the sample is added to a nucleic acid amplification mixture, it may be subjected to mechanical breakage, e.g., by bead beating or sonication, so that target nucleic acids may be more easily obtained from the sample. In some embodiments, extraction and purification of target nucleic acids is performed. Where a microfluidic cartridge is used, these steps may be performed in the microfluidic cartridge. In some embodiments, extraction and purification of target nucleic acids is performed in a sample preparation area of a microfluidic cartridge.

### D. Methods for Monitoring Liquid Filling of Reaction Chambers for Abnormalities

In an aspect, a method of monitoring for the introduction of one or more liquids into a reaction chamber includes: obtaining a first fluorescence measurement from the reaction chamber; introducing or attempting to introduce a first liquid into the reaction chamber; obtaining a second fluorescence measurement from the reaction chamber; determining a first value from the first fluorescence measurement and the second fluorescence measurement, the first value indicating a degree of change in fluorescence in the reaction chamber resulting from attempting to introduce a first liquid into the reaction chamber; and comparing the first value to a first predetermined threshold or range, wherein an abnormality is detected if the first value does not satisfy the first predetermined threshold or the first value is outside the first predetermined range.

In an aspect, a method of monitoring for the introduction of one or more liquids into a plurality of reaction chambers includes: obtaining a first fluorescence measurement from each of the reaction chambers; introducing or attempting to introduce a first liquid into each of the reaction chambers; obtaining a second fluorescence measurement from each of the reaction chambers; determining a first value from each of the plurality of first fluorescence measurements and each of the plurality of second fluorescence measurements, each first value indicating a degree of change in fluorescence in the corresponding reaction chamber resulting from introducing or attempting to introduce a first liquid into each of the reaction chambers; and comparing each of the first values to a first predetermined threshold or range, wherein an abnormality is detected if any one of the first values does not satisfy the first predetermined threshold or the first value is outside the first predetermined range.

In some embodiments, when attempting to introduce the first liquid into the reaction chamber, the attempt may or may not successfully introduce the first liquid into the reaction chamber.

In some embodiments, the method includes pumping or attempting to pump the first liquid through a tip into the reaction chamber. In some embodiments, the tip is a needle tip or a pipette tip.

In some embodiments, after attempting to introduce a first liquid, and before obtaining a second fluorescence measurement, a nucleic acid amplification reaction is initiated in the reaction chamber. In some embodiments, the nucleic acid amplification reaction includes initiating a denaturation or reverse transcription step.

In some embodiments, the first liquid includes one or more reagents, or a nucleic acid sample. In some embodiments, the second liquid includes one or more reagents, or a nucleic acid sample. In some embodiments, after obtaining a second fluorescence measurement from the reaction chamber, the method further includes i) attempting to introduce a second liquid into the reaction chamber. The second liquid may include one or more nucleic acid amplification reagents or a nucleic acid sample that is different from the nucleic acid amplification reagent(s) or nucleic acid sample of the first liquid. After the step attempting to introduce the second liquid, the method further includes obtaining a third fluorescence measurement from the reaction chamber. A second value may be determined from the second fluorescence measurement and the third fluorescence measurement, and the second value may be indicative of whether a change in fluorescence was caused by introducing the second liquid into the nucleic acid amplification reaction chamber. The method further includes iii) comparing the second value to a second predetermined threshold or range, wherein an abnormality is detected if the second value does not satisfy the second predetermined threshold or the second value is outside the second predetermined range.

In some embodiments, the first liquid and the second liquid together form a nucleic acid amplification mixture. In some embodiments, the nucleic acid amplification mixture is a thermocycled nucleic acid amplification mixture. In some embodiments, the nucleic acid amplification mixture is a fluorophore-containing nucleic acid amplification reaction mixture. For example, the fluorophore may be provided as a fluorophore-containing nucleic acid (e.g., labeled detection probe). In some embodiments, the fluorophore-containing nucleic acid reaction mixture is subjected to thermocycling. In some embodiments, the fluorophore-containing nucleic acid reaction mixture is a PCR reaction mixture or an RT-PCR reaction mixture.

### 1. Fluorescence Measurements

The methods include obtaining fluorescence measurements from reaction chambers, which may contain nucleic acid amplification reaction mixtures or components thereof or which may not yet have been filled with such mixtures or components. In some embodiments, fluorescence measurements are obtained from a plurality of reaction chambers. The source of fluorescence may be fluorophores, oligonucleotide probes, dNTPs, primers, and/or other reagents in the nucleic acid amplification reaction mixtures. Fluorescence may be measured in terms of relative fluorescence units (RFU). The measurement of fluorescence described herein differs from fluorescence measurements that may be performed in previous quantitative and/or real-time PCR methods in that the measurements herein are obtained before and/or during an early stage of nucleic acid amplification and do not reflect the production of an amplicon, but rather changes in the presence and composition of liquid in the chamber that result from liquid filling. Additionally, the measurements are used qualitatively, in the sense that they are evaluated with respect to one or more thresholds to give a qualitative output (such as a determination of whether there is an abnormality). Furthermore, the measurements may be made during a period before thermocycling commences. Thus, the present methods do not require fluorescence measurements during thermocycling to detect abnormalities.

In some embodiments, fluorescence measurements may be obtained before introducing or attempting to introduce a first liquid into the reaction chamber. In some embodiments, fluorescence measurements may be obtained when a reaction chamber is empty. In some embodiments, fluorescence measurements may be obtained after attempting to introduce a first liquid into the reaction chamber, and optionally before attempting to introduce a second liquid into the reaction chamber. In some embodiments, fluorescence measurements may be obtained after introducing or attempting to introduce a second liquid into the reaction chamber. In some embodiments, fluorescence measurements may be obtained when a reaction chamber contains a liquid. In some embodiments, fluorescence measurements are obtained at fixed time intervals.

In some embodiments, the first liquid and/or the second liquid includes one or more fluorophores. In some embodiments, the fluorophore is associated with oligonucleotide probe. In some embodiments, the oligonucleotide probe further includes a quencher.

In some embodiments, the first liquid and/or the second liquid does not include a fluorophore associated with an oligonucleotide. In these embodiments, inherent fluorescence of the one or more reagents and/or nucleic acid sample is measured. In some embodiments, the one or more reagents include dNTPs and/or one or more primers.

### a) First Fluorescence Measurement

In some embodiments, a first fluorescence measurement is obtained from a reaction chamber. A fluorescence measurement may be obtained with an optical detector, e.g. a fluorometer such as a photodiode or photomultiplier tube. In some embodiments, a nucleic acid amplification reaction mixture is not yet present in the chamber or not all components of a nucleic acid amplification reaction mixture are present in the chamber when the first measurement is obtained. In some embodiments, the first fluorescence measurement is obtained before introducing or attempting to introduce a first liquid into the reaction chamber.

In some embodiments, the first fluorescence measurement is an average or median of a plurality of individual measurements obtained from a nucleic acid amplification reaction chamber. In some embodiments, the plurality of individual measurements is obtained over a predetermined time period.

In some embodiments, a first fluorescence measurement is obtained from each of a plurality of reaction chambers. In some embodiments, the first fluorescence measurement is obtained before introducing or an attempt to introduce a first liquid into the plurality of reaction chambers. In some embodiments, after the first fluorescence measurement is obtained, the method further includes introducing or attempting to introduce a plurality of first liquids into the plurality of reaction chambers.

### b) Second Fluorescence Measurement

In some embodiments, a second fluorescence measurement is obtained. In some embodiments, the second fluorescence measurement is obtained during a denaturation or reverse transcription step. In some embodiments, the second fluorescence measurement is obtained before introducing or attempting to introduce a second liquid into the reaction chamber. In some embodiments, a nucleic acid amplification reaction is initiated after the second fluorescence measurement is obtained.

In some embodiments, the second fluorescence measurement is obtained after introducing or attempting to introduce a first liquid into the reaction chamber. The first liquid may be a nucleic acid amplification mixture or may contain one or more components of a nucleic acid amplification mixture. Additional components of the mixture may be provided after obtaining the second fluorescence measurement. In some embodiments, the second fluorescence measurement is an average or median of a plurality of individual measurements obtained from a nucleic acid amplification reaction chamber. In some embodiments, the plurality of measurements is obtained over a predetermined time period.

In some embodiments, a second fluorescence measurement is obtained from a plurality of reaction chambers. In some embodiments, the second fluorescence measurement is obtained after introducing or attempting to introduce a first liquid into the plurality of reaction chambers.

### c) Third Fluorescence Measurement

In some embodiments, a third fluorescence measurement is obtained. In some embodiments, a nucleic acid amplification reaction is initiated before the third fluorescence measurement is obtained. In some embodiments, the third fluorescence measurement is obtained during a denaturation or reverse transcription step.

In some embodiments, the third fluorescence measurement is obtained after introducing or attempting to introduce a second liquid into the reaction chamber. In some embodiments, the third fluorescence measurement is an average or median of a plurality of individual measurements obtained from a reaction chamber.

### 2. Values as Ratios of Fluorescence Measurements

### a) Determination of a First Value as a Ratio of the Second Fluorescence Measurement to the First Fluorescence Measurement

The methods include determining a first value from the first fluorescence measurement and the second fluorescence measurement, the first value indicating a degree of change in fluorescence in the reaction chamber resulting from introducing, or attempting to introduce, a first liquid of the one or more liquids into the nucleic acid amplification reaction chamber. In some embodiments, the degree of change of fluorescence may be zero or nearly zero in cases of certain abnormalities such as failure to introduce any a first liquid of the one or more liquids into the nucleic acid amplification reaction chamber.

The first value should reflect whether fluorescence changed between the first fluorescence measurement and the second fluorescence measurement due to the introduction of the first liquid or attempt to introduce the first liquid into the reaction chamber or the extent to which fluorescence changed between the first fluorescence measurement and the second fluorescence measurement due to the introduction of the first liquid or attempt to introduce the first liquid into the nucleic acid amplification reaction chamber. In some embodiments, the first value is determined as a ratio of the second fluorescence measurement to the first fluorescence measurement.

In some embodiments, where first and second fluorescence measurements were obtained from each of a plurality of chambers, the methods include determining a plurality of first values from the plurality of first fluorescence measurements and the plurality of second fluorescence measurements. the plurality of first values can indicate, for each of the plurality of chambers, whether a change in fluorescence was caused by introducing the first liquid into the nucleic acid amplification reaction chamber.

### b) Determination of a Second Value as a Ratio of the Third Fluorescence Measurement to the Second Fluorescence Measurement

In some embodiments, the methods include determining a second value from the fluorescence measurement and the third fluorescence measurement. The second value should reflect whether fluorescence changed between the second fluorescence measurement and the third fluorescence measurement due to the introduction of the second liquid or attempt to introduce the second liquid into the reaction chamber or the extent to which fluorescence changed between the second fluorescence measurement and the third fluorescence measurement due to the introduction of the second liquid or attempt to introduce the second liquid into the reaction chamber. In some embodiments, the second value is determined as a ratio of the third fluorescence measurement to the second fluorescence measurement. In some embodiments, the second value may indicate whether a change in fluorescence was caused by introducing the second liquid into the nucleic acid amplification reaction chamber. In some embodiments, the degree of change of fluorescence may be zero or nearly zero in cases of certain abnormalities such as failure to introduce any a second liquid of the one or more liquids into the nucleic acid amplification reaction chamber.

In some embodiments, where second and third fluorescence measurements were obtained from each of a plurality of chambers, the methods include determining a plurality of second values from a plurality of third fluorescence measurements and a plurality of second fluorescence measurements. In some embodiments, the plurality of second values can indicate, for each of the plurality of chambers, whether a change in fluorescence was caused by introducing the second liquid into the nucleic acid amplification reaction chamber.

### 3. Detection of Abnormalities

The methods include comparing the first value to a first predetermined threshold or range. In some embodiments, an abnormality is detected if the first value does not satisfy the first predetermined threshold. In some embodiments, an abnormality is detected if the first value is outside the first predetermined range.

In some embodiments, where a plurality of first values were determined, the method includes comparing the plurality of first values to a first predetermined threshold or range. In some embodiments, an abnormality is detected if any first value does not satisfy the first predetermined threshold or any first value is outside the first predetermined range.

In some embodiments, where a second value is determined, the method includes comparing the second value to a second predetermined threshold or range. In some embodiments, an abnormality is detected if the second value does not satisfy the second predetermined threshold or the second value is outside the second predetermined range.

In some embodiments, a predetermined threshold or range may be established from reference liquid filling runs. Reference liquid filling runs of nucleic acid amplification reaction chambers may include a plurality of successful and defective liquid handling events during a method described herein. Typically, as abnormal liquid filling event results in an error in the final volume and/or composition of a nucleic acid amplification reaction mixture, which can cause failure of the nucleic acid amplification reaction. The methods can thus enable detection of abnormalities in a nucleic acid amplification reaction chamber that may adversely affect the nucleic acid amplification reaction. The reference runs can include liquid handling events that were performed under different conditions. These conditions may include liquid filling abnormalities such as introducing bubbles into the nucleic acid amplification reaction chamber, filling a nucleic acid amplification reaction chamber with an incorrect liquid reagent, and/or filling a nucleic acid amplification reaction chamber with an inaccurate liquid volume. Inaccurate liquid volumes can include insufficient liquid volume, excessive liquid volume and/or no liquid volume transferred to a nucleic acid amplification reaction chamber. These conditions can include nucleic acid amplification reaction mixtures with one or more reagent changes that cause a reaction to fail. Fluorescence measurements taken at various points during these reference filling runs may be obtained. Ratios of these measurements may be used to determine a threshold and/or range of acceptable values. In some embodiments, the threshold or range is referred to as a "predetermined threshold" or "predetermined range" and is used to compare to values in the methods described herein.

Values in a certain range, or that satisfy or do not satisfy a certain threshold, may be indicative of one or more normal or abnormal liquid filling events. Exemplary thresholds are depicted, in FIGs 5 and 6.

In some embodiments, after attempting to introduce a first liquid into the nucleic acid amplification reaction chamber, no abnormality is detected when comparing the first value to a first predetermined threshold or range. In some embodiments, after the attempt to introduce a first liquid into the nucleic acid amplification reaction chamber, an abnormality is detected when comparing the first value to a first predetermined threshold or range.

In some embodiments, after the attempt to introduce a second liquid into the nucleic acid amplification reaction chamber, no abnormality is detected when comparing the second value to a second predetermined threshold or range. In some embodiments, after the attempt to introduce a second liquid into the nucleic acid amplification reaction chamber, an abnormality is detected when comparing the second value to a second predetermined threshold or range.

### 4. Liquid Filling Abnormalities

To detect liquid filling abnormalities in a nucleic acid amplification reaction chamber, in some embodiments, a first value of the methods herein is compared to a first predetermined threshold or range. In some embodiments, a second value of the methods herein is compared to a second predetermined threshold or range.

In some embodiments, an abnormality is detected if any first value does not satisfy the first predetermined threshold. In some embodiments, an abnormality is detected if any first value is outside the first predetermined range. In some embodiments, an abnormality is detected if any second value does not satisfy the second predetermined threshold. In some embodiments, an abnormality is detected if any second value is outside the second predetermined range.

In some embodiments, the abnormality includes defective liquid handling. In some embodiments, defective liquid handling may include introducing bubbles, inaccurate liquid volumes, and/or incorrect liquid reagents (such as buffers, salts, and other aqueous solutions) into a nucleic acid amplification reaction chamber, thereby causing an abnormality in the nucleic acid amplification reaction.

In some embodiments, when attempting to introduce a liquid into a nucleic acid amplification reaction chamber, an incorrect liquid or an incorrect volume may be used. In such instances, an abnormality is detected.

In some embodiments, when attempting to introduce a first liquid into the reaction chamber, the first liquid is introduced. In these embodiments, no abnormality is detected. In some embodiments, when attempting to introduce a first liquid into the reaction chamber, the first liquid is not introduced. In these embodiments, an abnormality is detected.

In some embodiments, when attempting to introduce a first volume of the first liquid into the reaction chamber, a second volume of the first liquid is introduced instead. In some embodiments, the second volume less than the first volume, and an abnormality is detected.

In some embodiments, when attempting to introduce a first volume of a first liquid into the reaction chamber, the first volume is introduced into the nucleic acid amplification reaction chamber within a tolerance of 10%, 5%, 2%, 1%, or 0.5%, of that first volume. In these embodiments, no abnormality is detected.

In some embodiments, when attempting to introduce a second liquid into the reaction chamber, the second liquid is not introduced. In these embodiments, an abnormality is detected. In some embodiments, when attempting to introduce a first volume of the second liquid into the reaction chamber, a second volume of the second liquid is introduced instead. In some embodiments, the second volume is less than the first volume. In these embodiments, an abnormality is detected.

In some embodiments, when attempting to introduce a first volume of the second liquid into the reaction chamber, the first volume is introduced into the reaction chamber within a tolerance of 10%, 5%, 2%, 1%, or 0.5% of that first volume. In these embodiments, no abnormality is detected.

### E. Systems and Computer-Readable Media

Disclosed herein are systems. A system refers to a device or an apparatus that may be used to perform, monitor and/or analyze liquid filling in reaction chambers as described herein. Provided herein is a nucleic acid amplification system including: (1) a docking station configured to receive a vessel including at least one reaction chamber; (2) a fluorometer configured to measure fluorescence in the reaction chamber; and (3) a processor operably linked to the fluorometer and a memory. The memory linked to the processor includes instructions that when executed by the processor cause the system to perform a method of monitoring for the introduction of one or more liquids into the reaction chamber for an abnormality. The method of monitoring includes the following steps: obtaining a first fluorescence measurement from the nucleic acid amplification reaction chamber; introducing, or attempting to introduce a first liquid into the reaction chamber; , obtaining a second fluorescence measurement from the reaction chamber; and determining a first value from the first fluorescence measurement and the second fluorescence measurement, the first value indicating a degree of change in fluorescence in the nucleic acid amplification reaction chamber resulting from attempting to introduce a first liquid into the reaction chamber; and comparing the first value to a first predetermined threshold or range. When comparing the first value to a first predetermined threshold or range, an abnormality is detected if the first value does not satisfy the first predetermined threshold or the first value is outside the first predetermined range. In some embodiments, the nucleic acid amplification system includes instructions that when executed by the processor cause the system to perform methods of monitoring or the introduction of one or more liquids into a reaction chamber as described herein.

In some embodiments, the system is a nucleic acid amplification system. In some embodiments, the system includes a docking station to receive one or more vessels. An exemplary nucleic acid amplification, system is shown in Figures 7-9. Figure 7 is an exemplary system capable of nucleic acid amplification loaded with a microfluidic cartridge in its docking station. An exemplary system may include a heat sink, a fluorometer, and a press/ heater used for interacting and measuring a sample loaded in a vessel. Figure 8 is a side view of the exemplary system shown in Figure 7. The side view shows the fiber optics that run from the cartridge to the fluorometer as well as a detector, bore and aluminum block used for taking fluorescence measurements. Figure 9 shows a cutaway view of the nucleic acid amplification system docking station and a microfluidic cartridge of Figures 7 and 8. The cutaway view shows internal components such as the thermal cycler, thermal block, array detector, rotary valve system and pistons useful for moving and analyzing liquids in different functional areas of the cartridge, as well as subjecting the vessel (e.g., a'microfluidic cartridge) to nucleic acid amplification conditions.

In some embodiments, the vessel is a multi-chambered receptacle, or the nucleic acid amplification chamber is contained within a multi-chambered receptacle.

In some embodiments, the system includes a temperature controller and is capable of providing or transferring heat to nucleic acid amplification reaction chambers via one or more heating elements. In some embodiments, the system is programmable such that it can hold nucleic acid amplification reaction chambers at set temperatures for different lengths of time.

In some embodiments, the system includes a fluorometer configured to measure and record the fluorescence of one or more fluorophores in the nucleic acid amplification reaction chamber. When a vessel is present in the system, e.g., in a docking station, the fluorometer is disposed in proximity to a position occupied by or to be occupied by a nucleic acid amplification reaction mixture (i.e., a functional area of the vessel in optical communication with the fluorometer).

In some embodiments, the system may also include sensors to monitor and/or estimate liquid handling of the nucleic acid amplification reaction chambers. In some embodiments, a sensor may be a thermal sensor, a capacitive sensor, or an infrared sensor. The nucleic acid amplification system may include a processor operably linked to the fluorometer and a memory. The memory includes instructions that when executed by the processor, can cause the system to perform methods of monitoring a nucleic acid amplification reaction chambers for an abnormality described herein.

In some embodiments, in the event that an abnormality is detected, the nucleic acid amplification system provides one or more alarms. Alarms may be provided to indicate liquid filling abnormalities. Examples of alarms include visual notifications and audio notifications. In some examples, when an alarm is provided, the nucleic acid amplification system pauses a method of nucleic acid amplification, which may include adjusting the heating element to about 4°C and holding that temperature for a period of time.

In some embodiments, the system is configured not to subject the reaction chamber to nucleic acid amplification conditions if an abnormality is detected and/or is configured to subject the reaction chamber to nucleic acid amplification conditions only if an abnormality is not detected.

Also disclosed herein are computer-readable media. In some embodiments, a computer-readable medium includes instructions that when executed by a processor of a system, cause the system to perform a method of monitoring for the introduction of one or more liquids into a reaction chamber described herein, e.g., including the following steps: obtaining a first fluorescence measurement from the reaction chamber; introducing or attempting to introduce a first liquid into the reaction chamber, the first liquid including one or more reagents or a nucleic acid sample; obtaining a second fluorescence measurement from the reaction chamber; determining a first value from the first fluorescence measurement and the second fluorescence measurement, the first value indicating a degree of change in fluorescence in the reaction chamber resulting from obtaining a second fluorescence measurement from the reaction chamber; and comparing the first value to a first predetermined threshold or range, wherein an abnormality is detected if the first value does not satisfy the first predetermined threshold or the first value is outside the first predetermined range.

In some embodiments, the computer-readable medium includes instructions for performing methods of monitoring liquid filling of a reaction chamber as described herein. In some embodiments, these instructions are executed by a processor of the system as described herein.

In some embodiments, the method includes subjecting the reaction mixture to nucleic acid amplification conditions only if an abnormality is not detected. In some embodiments, the nucleic acid amplification conditions include thermocycling. In some embodiments, the method includes discontinuing a nucleic acid amplification reaction if an abnormality is detected.

### EXAMPLES

### Example 1

This Example describes performing liquid filling runs of nucleic acid amplification chambers under normal conditions and under conditions with liquid filling abnormalities. This collection of runs provided the basis for predetermining a threshold and/or range to compare first or second values in the methods described herein.

Reaction mixtures including Amplidiag^{®} Multiplex PCR Master Mix; a nucleic acid template; forward and reverse primers; and fluorescent probes were prepared.

The reaction mixtures were subjected to amplification in a Novodiag^{®} thermal cycler. Attempts to introduce a first and second liquid were made as indicated by the arrows in Figure 3. Fluorescence measurements from the nucleic acid amplification reaction chamber were obtained at various points during these filling runs, including before and after liquid injections into the nucleic acid amplification reaction chamber. (FIG. 1 and FIG. 3, indicated by horizontal bars).

Ratios of fluorescence measurements obtained before and after liquid injections were plotted and thresholds were determined (FIG. 2 and FIGs. 4-6).

Values in a certain range, or that satisfy or do not satisfy a certain threshold, may be indicative of one or more liquid filling events, e.g. a failed run, a missing injection of a liquid (a type of failed liquid filling attempt), a dry chamber, and/or standard liquid filling.

As shown in Figure 5, thresholds may be determined by plotting ratios of fluorescence measurements obtained before and after attempted liquid filling attempts. The y-axis representing the ratio of measurements obtained before attempting to introduce the first liquid and after attempting to introduce the second liquid. The x-axis representing the ratio of measurements obtained after attempting to introduce the first liquid and before attempting to introduce the first liquid. Thresholds are indicated by solid black lines and can be determined according to user and or equipment parameters during liquid filling runs. As shown in Figure 5, values above threshold 2 (thr2; y-axis), and below threshold 1 (thr1; x-axis), indicate reaction mixtures with a failed second liquid filling attempt. Values below threshold 2 (thr2; y-axis) and below threshold 1 (thr1; x-axis), indicate a failed run or a dry chamber (i.e., no liquid filling attempts were successful). Values above threshold 3 (thr3; y-axis) and above threshold 1 (thr1; x-axis), indicate reaction mixtures with successful first and second liquid filling attempts (i.e., standard filling). Values below threshold 3 (thr3; y-axis) and above threshold 1 (thr1; x-axis) indicate reaction mixtures with a failed first liquid filling attempt (i.e., missing a first injection).

Alternatively, thresholds may be determined by plotting ratios of fluorescence measurements obtained after attempted liquid filling attempts. As shown in Figure 6, the y-axis represents the ratio of measurements obtained after attempting to introduce the first liquid and after attempting to introduce the second liquid, while the x-axis represents the number of runs. Thresholds are indicated by solid black lines and can be determined according to user and or equipment parameters during liquid filling runs. In this example, values above threshold 1 (thr1) indicate a failed first liquid filling attempt. Values between threshold 1 (thr1) and threshold 2 (thr2) indicate a failed run or dry chamber. Values between threshold 2 (thr2) and threshold 3 (thr3) indicate successful first and second liquid filling attempts (i.e., standard filling). Values below threshold 3 (thr3) indicate a failed second liquid filling attempt.

**Results:** The fluorescence ratios for a plurality of runs are plotted in Figure 2 and Figures 3-6. Most runs with fluidic errors have fluorescence ratios that are distinct from normal runs. Thus, the method was effective at detecting liquid filling abnormalities errors based on fluorescence data obtained during reference liquid filling runs of nucleic acid amplification chambers under normal conditions and under conditions with liquid filling abnormalities in a thermocycled amplification reaction.

### EQUIVALENTS

The foregoing written specification is considered to be sufficient to enable one skilled in the art to practice the embodiments. The foregoing description and Examples detail certain embodiments and describes the best mode contemplated by the inventors. It will be appreciated, however, that no matter how detailed the foregoing may appear in text, the embodiment may be practiced in many ways and should be construed in accordance with the appended claims and any equivalents thereof.

## Claims

1. A method of monitoring for the introduction of one or more liquids into a reaction chamber, the method comprising:
a) obtaining a first fluorescence measurement from the reaction chamber;
b) after step a), introducing, or attempting to introduce, a first liquid of the one or more liquids into the reaction chamber;
c) after step b), obtaining a second fluorescence measurement from the reaction chamber;
d) determining a first value from the first fluorescence measurement and the second fluorescence measurement, the first value indicating a degree of change in fluorescence in the reaction chamber resulting from step b); and
e) comparing the first value to a first predetermined threshold or range, wherein an abnormality is detected if the first value does not satisfy the first predetermined threshold or the first value is outside the first predetermined range, optionally wherein the abnormality is defective liquid handling.

2. The method of claim 1, wherein introducing, or attempting to introduce, the first liquid into the reaction chamber comprises pumping, or attempting to pump, the first liquid through a tip into the reaction chamber, optionally wherein the tip is a needle tip or a pipette tip.

3. The method of claim 1 or 2, wherein the first value is determined as a ratio of the second fluorescence measurement to the first fluorescence measurement, optionally wherein the first fluorescence measurement is an average or median of a plurality of individual measurements acquired in step a) and/or wherein the second fluorescence measurement is an average or median of a plurality of individual measurements acquired in step c).

4. The method of any one of the preceding claims, wherein after step b) and before step c), a nucleic acid amplification reaction is initiated in the reaction chamber, optionally wherein initiation of the nucleic acid amplification reaction comprises initiating a denaturation or reverse transcription step, and optionally wherein the second fluorescence measurement is obtained during the denaturation or reverse transcription step.

5. The method of any one of the preceding claims, wherein the first liquid is located in the nucleic acid amplification area during step c) if it was successfully introduced in step b), optionally wherein the first liquid is located in the nucleic acid amplification area during step c).

6. The method of any one of the preceding claims, wherein a first fluorescence measurement is obtained from each of a plurality of reaction chambers in step a).

7. The method claim 6, wherein
- step b) comprises introducing, or attempting to introduce, a first liquid into each of the plurality of reaction chambers in step b);
- step c) comprises obtaining a second fluorescent measurement from each of the plurality of reaction chambers in step c);
- step d) comprises determining a first value from each of the plurality of first fluorescence measurements and each of the plurality of second fluorescence measurements, each first value indicating a degree of change in fluorescence in the corresponding reaction chamber resulting from step b); and/or
- step e) comprises comparing each first value to a first predetermined threshold or range, wherein an abnormality is detected if any first value does not satisfy the first predetermined threshold or any first value is outside the first predetermined range.

8. The method of any one of the preceding claims, wherein the first liquid comprises a fluorophore, optionally wherein the fluorophore is associated with an oligonucleotide probe, and optionally wherein the oligonucleotide probe further comprises a quencher; or wherein the first liquid does not comprise a fluorophore associated with an oligonucleotide, inherent fluorescence of the one or more reagents or a sample is measured, and optionally wherein the one or more reagents comprise dNTPs and/or one or more primers..

9. The method of any one of the preceding claims, wherein the method further comprises, after step c):
i) introducing, or attempting to introduce, a second liquid into the reaction chamber, optionally wherein the second liquid comprises one or more nucleic acid amplification reagents or a sample different from the one or more nucleic acid amplification reagents or nucleic acid sample of the first liquid;
ii) after step i), obtaining a third fluorescent measurement from the reaction chamber;
iii) determining a second value from the second fluorescent measurement and the third fluorescent measurement, the second value indicating a degree of change in fluorescence in the reaction chamber resulting in step i), optionally wherein the second value is determined as a ratio of the third fluorescence measurement to the second fluorescence measurement, and optionally wherein the third fluorescence measurement is an average or median of a plurality of individual measurements acquired in step ii).; and
iv) comparing the second value to a second predetermined threshold or range, wherein an abnormality is detected if the second value does not satisfy the second predetermined threshold or the second value is outside the second predetermined range.

10. The method of claim 9, wherein after step i) and before step ii), a nucleic acid amplification reaction is initiated; optionally wherein initiation of the nucleic acid amplification reaction comprises initiating a denaturation or reverse transcription step, and optionally wherein the third fluorescence measurement is obtained during the denaturation or reverse transcription step.

11. The method of claim 9 or 10, wherein the first liquid and the second liquid together form a nucleic acid amplification reaction mixture, and optionally wherein the nucleic acid amplification reaction mixture is a thermocycled nucleic acid amplification reaction mixture.

12. The method of any one of claims 9-11, wherein
- the second liquid is introduced into the reaction chamber and no abnormality is detected in step iii);
- the second liquid is not introduced into the reaction chamber and an abnormality is detected in step iii);
- step i) comprises attempting to introduce a first volume of the second liquid into the reaction chamber but a second volume of the second liquid is introduced into the reaction chamber, the second volume being less than the first volume, and an abnormality is detected in step iv); or
- step i) comprises attempting to introduce a first volume of the second liquid into the reaction chamber and the first volume within a tolerance of 10%, 5%, 2%, 1%, or 0.5% is introduced into the reaction chamber, and no abnormality is detected in step iii).

13. The method of any one of the preceding claims, wherein
- the first liquid is introduced into the reaction chamber and no abnormality is detected in step e);
- the first liquid is not introduced into the reaction chamber and an abnormality is detected in step e);
- step b) comprises attempting to introduce a first volume of the first liquid into the reaction chamber but a second volume of the first liquid is introduced into the reaction chamber, the second volume being less than the first volume, and an abnormality is detected in step e); or
- step b) comprises attempting to introduce a first volume of the first liquid into the reaction chamber and the first volume is introduced into the reaction chamber within a tolerance of 10%, 5%, 2%, 1%, or 0.5%, and no abnormality is detected in step e).

14. The method of any one of claims 1-11 and 13, further comprising subjecting the reaction chamber to nucleic acid amplification conditions only if an abnormality is not detected, and optionally comprising discontinuing a nucleic acid amplification reaction if an abnormality is detected.

15. The method of claim 13, wherein the nucleic acid amplification conditions comprise thermocycling.

16. A system comprising:
a docking station configured to receive a vessel comprising a reaction chamber;
a fluorometer configured to measure fluorescence in the reaction chamber;
and a processor operably linked to the fluorometer and a memory;
the memory comprising instructions that when executed by the processor cause the system to perform a method of monitoring for the introduction of one or more liquids into the reaction chamber, the method comprising:
a) obtaining a first fluorescence measurement from the reaction chamber;
b) after step a), introducing, or attempting to introduce, a first liquid of the one or more liquids into the reaction chamber;
c) after step b), obtaining a second fluorescence measurement from the reaction chamber;
d) determining a first value from the first fluorescence measurement and the second fluorescence measurement, the first value indicating a degree of change in fluorescence in the reaction chamber resulting from step b); and
e) comparing the first value to a first predetermined threshold or range, wherein an abnormality is detected if the first value does not satisfy the first predetermined threshold or the first value is outside the first predetermined range.

17. The system of claim 16, wherein the vessel is a multi-chambered receptacle or the reaction chamber is contained within a multi-chambered receptacle, optionally wherein the reaction chamber is contained within a microfluidic cartridge, and optionally wherein the microfluidic cartridge comprises:
a plurality of functional areas comprising a sample preparation area, a nucleic acid amplification area, and a waste area;
a central distribution hub; and
a pump, a plurality of valves, and a fluidic network of microchannels connecting the functional areas to the hub;
wherein the pump, plurality of valves, and fluidic network of microchannels can drive movement of a fluid from a first functional area through the central distribution hub to a second functional area of the plurality of functional areas;
and the reaction chamber is or is within the nucleic acid amplification area.

18. The system of claim 16, wherein the nucleic acid amplification reaction mixture is contained within a microfluidic cartridge, optionally wherein the microfluidic cartridge comprises:
- a plurality of functional areas comprising a sample preparation area, a nucleic acid amplification area, and a waste area;
- a central distribution hub; and
- a pump, a plurality of valves, and a fluidic network of microchannels connecting the functional areas to the hub;
- wherein the pump, plurality of valves, and fluidic network of microchannels can drive movement of a fluid from a first functional area through the central distribution hub to a second functional area of the plurality of functional areas, and optionally wherein the nucleic acid amplification reaction mixture is located in the nucleic acid amplification area during step ii).

19. The system of any one of claims 16 configured to perform the method of claims 1-11 and 13, wherein the system is configured not to subject the reaction chamber to nucleic acid amplification conditions if an abnormality is detected and/or is configured to subject the reaction chamber to nucleic acid amplification conditions only if an abnormality is not detected, and optionally wherein the nucleic acid amplification conditions comprise thermocycling.

20. The system of any one of claims 16-18, wherein the memory comprises instructions that when executed by the processor cause the system to perform the method according to any one of claims 1 to 15.

21. A computer-readable medium comprising:
instructions that when executed by a processor of a system cause the system to perform a method of monitoring for the introduction of one or more liquids into a reaction chamber, the method comprising:
a) obtaining a first fluorescence measurement from the reaction chamber;
b) after step a), introducing, or attempting to introduce, a first liquid of the one or more liquids into the reaction chamber;
c) after step b), obtaining a second fluorescence measurement from the amplification reaction chamber;
d) determining a first value from the first fluorescence measurement and the second fluorescence measurement, the first value indicating a degree of change in fluorescence in the reaction chamber resulting from step b); and
e) comparing the first value to a first predetermined threshold or range, wherein an abnormality is detected if the first value does not satisfy the first predetermined threshold or the first value is outside the first predetermined range.

22. The computer-readable medium of claim 21 comprising instructions that when executed by a processor of a system cause the system to perform the method of any one of claims 1 to 15.
